# EUROPÄISCHE PATENTSCHRIFT

(11) **EP 1 211 248 B1**
(45) Veröffentlichungstag und Bekanntmachung des Hinweises auf die Patenterteilung: **24.09.2003**
(21) Anmeldenummer: 01117263.2
(22) Anmeldetag: 17.07.2001
(51) Int. Cl.: C07D 239/54, C08K 5/3462

(54) **Verfahren zur Herstellung von Aminouracilen**
Process for the preparation of aminouraciles
Procédé de préparation d' aminouraciles

(30) Priorität: 02.12.2000 DE 10060094; 24.03.2001 DE 10114454
(43) Veröffentlichungstag der Anmeldung: 05.06.2002
(73) Patentinhaber: Cognis Deutschland GmbH & Co. KG, 40589 Düsseldorf (DE)
(72) Erfinder: Daute, Peter, Dr, 27616 Beverstedt (DE); Wedl, Peter, 27568 Bremerhaven (DE); Picard, Ralf, 27572 Bremerhaven (DE); Klamann, Jörg-Dieter, Dr,, 27574 Bremerhaven (DE); Fleder, Thomas, 27612 Loxstedt (DE); Leisentritt, Stephan, 27578 Bremerhaven (DE)

(56) Entgegenhaltungen:
- WO-A-00/68207
- DD-A- 140 878
- F.FÜLLE: "A NOVEL RING CLOSURE REACTION FOR THE PREPARATION OF 6-AMINOURACILS" HETEROCYCLES., Bd. 53, Nr. 2, 2000, Seiten 347-351, XP002191739 ELSEVIER SCIENCE PUBLISHERS B.V. AMSTERDAM., NL ISSN: 0385-5414
- CHEMICAL ABSTRACTS, vol. 88, no. 28, 1978 Columbus, Ohio, US; abstract no. 152547p, ABRAMOVA,E.: "PREP. OF 1,3-DIMETHYL-4-AMINO-5-NITROSOURACIL." Seite 603; Spalte 2; XP002191740 & KHIM.-FARM. ZH., Bd. 12, Nr. 1, 1978, Seiten 96-8, USSR

## Beschreibung

### Gebiet der Erfindung

Die Erfindung betrifft ein Verfahren zur Herstellung von Aminouracilen sowie die Verwendung der so hergestellten Aminouracile zur Stabilisierung von halogenhaltigen organischen Kunststoffen.

### Stand der Technik

Halogenhaltige Kunststoffe oder daraus hergestellte Formmassen neigen bekanntermaßen zu Abbau- beziehungsweise Zersetzungsreaktionen, wenn sie thermischer Belastung ausgesetzt sind oder mit energiereicher Strahlung, zum Beispiel Ultraviolettlicht, in Kontakt kommen.

Zur Stabilisierung von PVC bei der Verarbeitung werden meist metallhaltige Stabilisatoren auf Basis von Pb, Ba, Cd, Sn, Ca und Zn eingesetzt. Bereits 1940 wurden Harnstoffderivate wie z.B. Diphenylthioharnstoff zur Stabilisierung von PVC vorgeschlagen (vergleiche: **Gächter/Müller, "Kunststoff-Additive", Carl Hanser Verlag 1989, S 312)** Diese Verbindungen werden meist in Kombination mit metallhaltigen Stabilisatoren eingesetzt, da sie alleine in aller Regel keine ausreichende Langzeitstabilisierung ergeben.

**EP-A-768 336** beschreibt Stabilisatorkombinationen für chlorhaltige Polymere, insbesondere für Polyvinylchlorid (PVC) mit einem Gehalt an speziellen heterocyclischen Verbindungen und zwar substituierten Uracilderivaten. Darüber hinaus enthalten diese Kombinationen zwingend mindestens eine Verbindung, die aus gewählt ist aus der Gruppe der Perchlorat-Verbindungen, Glycidylverbindungen, beta-Diketone bzw. -Ester, Dihydropyridine und Polyhydropyridine, sterisch gehinderte Amine, Zeolithe, Hydrotalcite, Dawsonite, Alkali- und Erdalkaliverbindungen, Antioxidantien und Gleitmitte sowie Organozinnstabilisatoren.

**EP-A-930 332** beschreibt Stabilisatorkombinationen für chlorhaltige Polymere, insbesondere für Polyvinylchlorid (PVC) mit einem Gehalt an speziellen heterocyclischen Verbindungen und zwar substituierten Uracilderivaten. Darüber hinaus enthalten diese Kombinationen zwingend mindestens eine Verbindung, die ausgewählt ist aus der Gruppe der Katoite, Calcium-Hydroxy-Aluminium-hydrogenphosphite, Aluminiumhydroxide, Lithium-Schichtgitterverbindungen.

### Beschreibung der Erfindung

Aufgabe der vorliegenden Erfindung war es, Aminouracile bereitzustellen, die sich durch verbesserte Eigenschaften hinsichtliche der Stabilisierung von halogenhaltigen organischen Kunststoffen, insbesondere PVC, gegen thermischen und/oder photochemischen Abbau auszeichnen.

Gegenstand der vorliegenden Erfindung ist ein **Verfahren** zur Herstellung von Aminouracilen der Formel (ii) 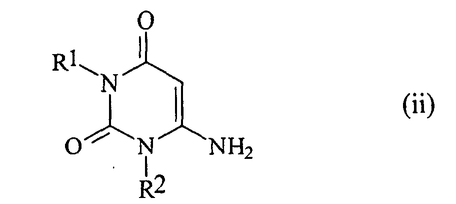 worin die Reste R¹ und R² unabhängig voneinander jeweils Wasserstoff oder einen unverzweigten oder verzweigten, linearen oder cyclischen Alkylrest mit 1 bis 18 C-Atomen oder einen Arylrest mit 6 bis 18 C-Atomen, der gegebenenfalls durch ein oder mehrere Alkylreste mit jeweils 1 bis 6 C-Atomen substituiert sein kann, bedeuten, durch Umsetzung der entsprechenden Cyanacetylharnstoffe der Formel (i) 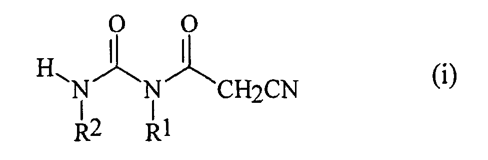 worin die Reste R¹ und R² die oben genannte Bedeutung haben,
mit basischen Verbindungen, wobei man die Umsetzung in einer Matrix durchführt, mit der Maßgabe, daß diese Matrix eine Zusammensetzung darstellt, die ein oder mehrere Verbindungen enthält, die ausgewählt sind aus der Gruppe der Kunststoffadditive, der Kunststoffstabilisatoren und der halogenhaltigen organischen Kunststoffe.

In einer Ausführungsform bedeuten die Reste **R**^{**1**} **und R**^{**2**} unabhängig voneinander jeweils einen unverzweigten oder verzweigten, linearen oder cyclischen Alkylrest mit 1 bis 18 C-Atomen .
In einer bevorzugten Ausführungsform haben die Reste R¹ und R² die Bedeutung Methyl. Dies bedeutet, daß man in diesem Falle als Verbindung (i) N,N'-Dimethyl-N-cyanacetylharnstoff einsetzt, wobei im Zuge des erfindungsgemäßen Verfahrens Dimethylaminouracil entsteht:

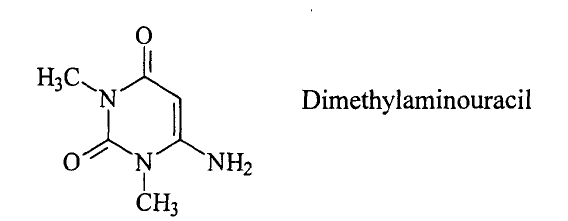

Die **Cyanacetylharnstoffe (i)** werden vorzugsweise in einer Menge von 0,01 bis 50 Gew.-% - bezogen auf die Gesamtheit der Matrix - eingesetzt. Dabei ist der Bereich von 0,05 bis 30 Gew.-% und insbesondere 1,0 bis 20 Gew.-% bevorzugt.

Die Wahl der **basischen Verbindungen** unterliegt an sich keinen Einschränkungen. In funktionaler Hinsicht kommen alle basischen Verbindungen in Betracht, die in der Lage sind, die Umwandlung der Verbindungen (i) in die Verbindungen (ii) zu bewirken. Im Rahmen der vorliegenden Erfindung ist es jedoch bevorzugt, die basischen Verbindungen aus der Gruppe der Alkali- und Erdalkalihydroxide zu wählen. Dabei sind wiederum die Erdalkalihydroxide, insbesondere Calciumhydroxid, ganz besonders bevorzugt. In einer Ausführungsform setzt man die basischen Verbindungen in einer Menge von mindestens 0,1 Gew.-% - bezogen auf die Gesamtheit der Cyanacetylharnstoffe (i) - ein. Als obere Grenze für die Menge an den basischen Verbindungen ist ein Wert von etwa 5000 Gew.-% - bezogen auf die Gesamtheit der Cyanacetylharnstoffe (i) - anzusehen. Ein Gewichtsverhältnis von (i) zu basischen Verbindung von 1:0,1 bis 1:100 ist bevorzugt.

Die **Durchführung** des erfindungsgemäßen Verfahrens kann auf verschiedene Weise erfolgen. Eine erste Ausführungsform besteht darin, die basischen Verbindungen zu einer Mischung aus Matrix und den Cyanacetylharnstoffen (i) zuzudosieren. Dies kann auf verschiedene Weise erfolgen. Beispiele hierfür sind:
- Zugabe der basischen Verbindungen in Form einer Lösung, insbesonderer einer wäßrigen Lösung.
- Zugabe der basischen Verbindungen als Feststoff.

Eine andere Ausführungsform besteht darin, die Cyanacetylharnstoffe (i) mit den gewünschten Komponenten der Matrix abzumischen und diese Mischung in Form einer Schmelze zu den vorgelegten basischen Verbindungen zuzudosieren. Dabei können die Cyanacetylharnstoffe beim Einbringen in die Matrix in Form einer Lösung oder als Schmelze eingesetzt werden.

Ein besonderer Vorzug des erfindungsgemäßen Verfahrens besteht darin, daß er keine speziellen Reaktionsapparaturen benötigt, sondern in beliebigen Mischaggregaten durchgeführt werden kann. Beispiele für besonders geeignete Systeme sind hierbei einfache Mischer sowie Extruder.

Die Durchführung der Umsetzung der Verbindungen (i) mit den basischen Verbindungen unterliegt an sich keinen Beschränkungen. In einer Ausführungsform erfolgt sie, indem man die Komponente (i) während der Extrusion der Matrix zudosiert. In einer anderem Ausführungsform wird die Komponente (i) der Matrix in einem Mischer zudosiert.

Die Reaktionstemperatur ist beim erfindungsgemäßen Verfahren an sich nicht kritisch. Insbesondere wählt man einen Temperaturbereich von 10 bis 180 °C. Es kann gewünscht sein, das Verfahren bei sehr mäßigen Temperaturen durchzuführen, etwa bei Reaktionstemperaturen im Bereich von 10 bis 100 °C und insbesondere im Bereich von 10 bis 50 °C und ganz besonders im Bereich von etwa 20 bis 30 °C. In anderen Ausführungsformen, insbesondere wenn man die Matrix in Form einer Schmelze einsetzt, kann eine höhere Reaktionstemperatur gewünscht sein; in diesen Fällen wählt man mindestens die Schmelztemperatur der Matrix. Vorzugsweise setzt man dabei eine Matrix ein, deren Schmelztemperatur unterhalb von 100 °C liegt und wählt die Reaktionstemperatur dann so, daß sie 10 bis 70 °C oberhalb dieser Schmelztemperatur liegt.

Das erfindungsgemäße Verfahren erfordert nur sehr kurze Reaktionszeiten. Vorzugsweise liegen diese unterhalb von 60 Minuten, insbesondere im Bereich von 2 bis 20 Minuten. Wenn für innige Durchmischung gesorgt wird - vergleiche etwa Beispiel 1 der vorliegenden Anmeldung - können bereits Reaktionszeiten von etwa 5 Minuten ausreichend sein.

Durch das erfindungsgemäße Verfahren, dessen Kern darin besteht, daß die durch die basischen Verbindungen induzierte Umwandlung der Verbindungen (i) in die Verbindungen (ii) in einer Matrix durchgeführt wird, wird ein Produkt erhalten, welches die hergestellten Verbindungen (ii) eingebunden in eine Matrix enthält. Überraschenderweise hat sich gezeigt, daß die Verbindungen (ii) in dieser Angebotsform eine **ausgezeichnete Wirkung** bei der Stabilisierung von halogenhaltigen organischen Kunststoffen gegen thermischen und/oder photochemischen Abbau aufweisen. Eingehende Untersuchungen der Anmelderin haben gezeigt, daß diese Wirkung signifikant besser ist, als wenn man entsprechende vorher separat auf konventionelle Art synthetisierte Verbindungen (ii) nachträglich in eine Matrix einbindet.

Die **Matrix** stellt eine Zusammensetzung dar, die ein oder mehrere Verbindungen enthält, die ausgewählt sind aus der Gruppe der Kunststoffadditive, der Kunststoffstabilisatoren und der halogenhaltigen organischen Kunststoffe. Beispiele für geeignete Substanzen der genannten Gruppe werden im Abschnitt "Komponenten der Matrix" gegeben. In einer speziellen Ausführungsform der Erfindung ist die Matrix frei von halogenhaltigen organischen Kunststoffen.

Ausdrücklich wird festgestellt, daß die Matrix definitionsgemäß niemals überwiegend Wasser sein kann. Sehr wohl aber ist es möglich, daß die Matrix - bedingt durch den Einsatz spezieller möglicher Komponenten - einen geringfügigen Anteil an Wasser enthalten kann. Dieser Anteil wir in aller Regel jedoch unterhalb von 10 Gew.-% und insbesondre unterhalb 5 Gew.-% - bezogen auf die gesamt Matrix - liegen.

Die nach dem erfindungsgemäßen Verfahren erhältlichen Zusammensetzungen, die die Komponente (ii) enthalten, eignen sich zur Stabilisierung von halogenhaltigen organischen Kunststoffen, wobei unter "Stabilisierung" insbesondere die Stabilisierung hinsichtlich Verfärbung bei thermischer und/oder photochemischer Belastung verstanden wird.

In einer bevorzugten Ausführungsform führt man das erfindungsgemäße Verfahren so durch, daß man als Matrixkomponente ein oder mehrere Gleitmittel einsetzt, diese Gleitmittel zunächst aufschmilzt und die Schmelze mit einer basischen Verbindung, insbesondere einem Alkali- oder Erdalkalihydroxid versetzt. Die Temperatur richtet sich dabei nach dem bzw. den eingesetzten Gleitmitteln. Es muß gewährleistet sein, daß das Gleitmittel in aufgeschmolzener, d.h. flüssiger Form vorliegt. Zu der genannten Mischung von Gleitmittel und basischer Verbindung - bei dieser Mischung handelt es sich in der Regel um eine Suspension - fügt man dann den Cyanoacetylharnstoff (i), vorzugsweise N,N'-Dimethyl-N-cyanoacetylhamstoff, zu; die Zugabe dieser Komponente kann in fester Form erfolgen, als Schmelze, oder - was besonders bevorzugt ist - in Form einer wäßrigen Lösung, wobei die Konzentration der wäßrigen Lösung an (i) insbesondere im Bereich von 30 bis 90 Gew.-% und besonders bevorzugt im Bereich von 50 bis 85 Gew.-% gewählt wird. Die Zugabe erfolgt vorzugsweise durch gleichmäßiges Zudosieren, im Falle einer wäßrigen Lösung von (i) etwa durch Zutropfen. Die so erhaltene Suspension wird anschließend temperiert, wobei diese Temperierung vorzugsweise so durchgeführt wird, daß man die Suspension auf eine Temperatur erwärmt, die 10 bis 70 °C über der Schmelztemperatur des eingesetzten Gleitmittels liegt. Die Temperierung erfüllt einerseits den Zweck, daß die gewünschte Reaktion zur Bildung der Verbindungen (ii) möglichst quantitativ abläuft, andererseits den Zweck, daß im System vorhandenes Wasser entfernt wird; letzteres kann durch Anlegen eines Vakuums noch unterstützt werden. Vorzugsweise wählt man die Temperatur im Bereich von 80 bis 150 °C. Sofern (i) in Form einer wäßrigen Lösung eingesetzt wurde, entfernt man das dadurch in das System eingetragene Wasser im Zuge der Temperierung, wobei man vorzugsweise ein Vakuum anlegt. Die Temperierungszeit wählt man vorzugsweise im Bereich von 1 bis 60 Minuten, insbesondere im Bereich von 2 bis 20 Minuten. Wie ausgeführt, setzt man (i) vorzugsweise in Form einer wäßrigen Lösung ein. Dabei stellt man insbesondere ein Gewichtsverhältnis von (i) zum Gleitmittel von 1:10 bis 1:1 ein. Desweiteren stellt man vorzugsweise ein Gewichtsverhältnis von (i) zu basischen Verbindung von 10:1 bis 1:10 ein. Im Hinblick auf die Auswahl des Gleitmittels bestehen an sich keine Beschränkungen. Diesbezüglich sei auf die Ausführungen im Abschnitt "Komponenten der Matrix" (vergleiche dort die Substanzen der Gruppe (d9) verwiesen. In einer bevorzugten Ausführungsform besteht die Matrix aus ein oder mehreren Gleitmitteln.

### Komponenten der Matrix

Wie bereits gesagt, stellt die **Matrix** eine Zusammensetzung dar, die ein oder mehrere Verbindungen enthält, die ausgewählt sind aus der Gruppe der Kunststoffadditive, der Kunststoffstabilisatoren und der halogenhaltigen organischen Kunststoffe. Hinsichtlich der Kunststoffadditive und - stabilisatoren sei ganz allgemein auf die eingangs zitierte Monographie von Gächter/Müller verwiesen.

In einer Ausführungsform enthält die Matrix als Komponente **Perchlorate a).** Perchlorate im Sinne der Erfindung sind Metallsalze und Ammoniumsalze der Perchlorsäure zu verstehen. Beispiele für erfindungsgemäß geeignete Perchlorate sind diejenigen der Formel M(ClO₄)ₙ, wobei M insbesondere für Ammonium, Li, Na, K, Mg, Ca, Sr, Zn, Al, La oder Ce steht. Der Index n ist entsprechend der Wertigkeit des Kations M 1, 2 oder 3.
Die Perchloratsalze können mit Alkoholen, etwa Polyolen, Cyclodextrinen, oder Ätheralkoholen bzw. Esteralkoholen komplexiert oder darin gelöst sein. Zu den Esteralkoholen sind auch die Polyolpartialester zu zählen. Bei mehrwertigen Alkoholen oder Polyolen kommen auch deren Dimere, Trimere, Oligomere und Polymere in Frage, wie Di-, Tri-, Tetra- und Polyglycole, sowie Di-, Tri- und Tetrapentaerythrit oder Polyvinylalkohol in verschiedenen Polymerisationsgraden. Im Hinblick auf Perchlorat-Alkohol-Komplexe seien ausdrücklich die dem Fachmann aus **EP-B-394 547**, Seite 3, Zeilen 37 bis 56 bekannten Typen miteinbezogen.
Die Perchloratsalze werden stets in Verbindung mit mindestens einer weiteren Matrix-Komponente eingesetzt. In diesem Zusammenhang können sie in verschiedenen gängigen Darreichungsformen eingesetzt werden, zum Beispiel als Salz oder Lösung in Wasser oder einem organischen Solvens als solches, bzw. aufgezogen auf ein Trägermaterial wie PVC, Ca-Silikat, Zeolithe oder Hydrotalcite, oder eingebunden durch chemische Reaktion in einen Hydrotalcit oder eine andere Schichtgitterverbindung. Als Polyolpartialether sind Glycerinmonoether und Glycerinmonothioether bevorzugt.
Die Perchlorate können sowohl einzeln als auch im Gemisch untereinander eingesetzt werden.
Die Perchlorate können in der Matrix in einer Menge von 0,1 bis 10, und insbesondere 0,5 bis 5 Gew.-Teilen, bezogen auf 100 Gew.-Teile der Matrix, enthalten sein. Mengen von etwa 3 Gew.-Teilen sind ganz besonders bevorzugt.

In einer Ausführungsform enthält die Matrix ein oder mehrere Verbindungen b). Diese werden ausgewählt aus der Gruppe folgender Substanzklassen:
b1) Zeolithe,
b2) kationischen Schichtgitterverbindungen,
b3) **C**alcium-**H**ydroxy-**A**luminium-(hydrogen)**p**hosphite (CHAP-Verbindungen)
b4) Katoite

Bei den **Zeolithen b1)** handelt es sich - wie dem Fachmann bekannt - um Alkali bzw. Erdalkalialumosilikate.

Sie können durch die allgemeine Formel (II)

M_{x/n}[(AlO₂)ₓ(SiO₂)_{y}]^{·}wH₂O (II)

beschrieben werden, worin
- n die Ladung des Kations M;
- M ein Element der ersten oder zweiten Hauptgruppe, wie Li, Na, K, Mg, Ca, Sr oder Ba;
- y:x eine Zahl von 0,8 bis 15, bevorzugt von 0,8 bis 1,2; und
- w eine Zahl von 0 bis 300, bevorzugt von 0,5 bis 30, ist.

Beispiele für Zeolithe sind Natriumalumosilikate der Formeln

Na₁₂ Al₁₂Si₁₂O₄₈. 27 H₂O [Zeolith A],

Na₆Al₆Si₆O₂₄. 2 NaX. 7,5 H₂O, X=OH, Halogen, ClO₄[Sodalith];

Na₆Al₆Si₃₀O₇₂. 24 H₂O;

Na₈Al₈Si₄₀O₉₆. 24 H₂O;

Na₁₆ Al₁₆Si₂₄O₈₀· 16 H₂O;

Na₁₆ Al₁₆Si₃₂O₉₆. 16 H₂O;

Na₅₆Al₅₆Si₁₃₆O₃₈₄. 250 H₂O [Zeolith Y],

Na₈₆Al₈₆Si₁₀₆O₃₈₄. 264 H₂O [Zeolith X];

oder die durch teilweisen bzw. vollständigen Austausch der Na-Atome durch Li-, K-, Mg-, Ca-, Sr- oder Zn-Atome darstellbaren Zeolithe wie

(Na,K)₁₀Al₁₀Si₂₂O₆₄. 20 H₂O;

Ca_{4,5}Na₃[(AlO₂)₁₂(SiO₂)₁₂]. 30 H₂O;

K₉Na₃[(AlO₂)₁₂(SiO₂)₁₂]. 27 H₂O.

Bevorzugte Zeolithe entsprechen den Formeln

Na₁₂Al₁₂Si₁₂O₄₈. 27 H₂O [Zeolith A],

Na₆Al₆Si ₆O₂₄. 2NaX. 7,5 H₂O, X = OH, Cl, ClO₄, 1/2CO₃[Sodalith]

Na₆Al₆Si₃₀ O₇₂ . 24 H₂O,

Na₈Al₈Si₄₀ O₉₆. 24 H₂O,

Na₁₆Al₁₆Si₂₄O₈₀. 16 H₂O,

Na₁₆Al₁₆Si₃₂O₉₆. 16 H₂O,

Na₅₆Al ₅₆Si₁₃₆O₃₈₄. 250 H₂O, [Zeolith Y]

Na₈₆Al₈₆Si₁₀₆O₃₈₄. 264 H₂O [Zeolith X]

und solche X- und Y-Zeolithe mit einem Al/ Si-Verhältnis von ca. 1:1 oder die durch teilweisen bzw. vollständigen Austausch der Na-Atome durch Li-, K-, Mg-, Ca-, Sr-, Ba- oder Zn-Atome darstellbaren Zeolithe wie

(Na,K)₁₀Al₁₀Si₂₂O₆₄. 20 H₂O.

Ca_{4,5}Na₃[(AlO₂)₁₂(SiO₂)₁₂]. 30 H₂O

K₉Na₃[(AlO₂)₁₂(SiO₂)₁₂]. 27 H₂O

Die angeführten Zeolithe können auch wasserärmer bzw. wasserfrei sein. Weitere geeigente Zeolithe sind:

Na₂O·Al₂O₃·(2 bis 5) SiO₂·(3,5 bis 10) H₂O [Zeolith P]

Na₂O·Al₂O₃·2 SiO₂·(3.5-10)H₂O (Zeolith MAP)

oder die durch teilweisen bzw. vollständigen Austausch der Na-Atome durch Li-, K-oder H-Atome darstellbaren Zeolithe wie

(Li,Na,K,H)₁₀Al₁₀ Si₂₂O₆₄. 20 H₂O.

K₉Na₃[(AlO₂)₁₂(SiO₂)₁₂]. 27 H₂O

K₄Al₄Si₄O₁₆ .6H₂O [Zeolith K-F]

Na₈Al₈Si₄₀O₉₆.24 H₂O Zeolith D, wie in Barrer et al.. J. Chem. Soc. 1952, 1561-71, und in US 2,950,952 beschrieben.

### Ferner kommen folgende Zeolithe in Frage:

K-Offretit, Zeolith R, Zeolith LZ-217, Ca-freier Zeolith LZ-218, Zeolith T, Zeolith LZ-220, Na₃ K₆Al₉Si₂₇O₇₂.21 H₂O [Zeolith L]; Zeolith LZ-211, Zeolith LZ-212, Zeolith O, Zeolith LZ-217, Zeolith LZ-219, Zeolith Rho, Zeolith LZ-214, Zeolith ZK-19, Zeolith W (K-M), Na₃₀Al₃₀Si₆₆O₁₉₂. 98 H₂O [Zeolith ZK-5, Zeolith Q]. Hinsichtlich dieser Zeolith-jTypen sei ausdrücklich auf die EP-A 768 336 und die dort zitierte Literatur verwiesen (vergliche EP-A 768 336 Seite 26, Zeilen 40 bis 54).

Besonders bevorzugt werden Zeolith P Typen der Formel II eigesetzt, worin x eine Zahl im Bereich von 2 bis 5 und y eine Zahl im Bereich von 3.5 bis 10 sind. Ganz besonders eignen sich Zeolith MAP der Formel II, worin x die Zahl 2 und y eine Zahl im Bereich von 3.5 bis 10 sind. Insbesondere handelt es sich um Zeolith Na-P, d.h. M steht für Na. Dieser Zeolith tritt im allgemeinen in den Varianten Na-P-1, NaP-2 und Na-P-3 auf, die sich durch ihre kubische, tetragonale oder orthorhombische Struktur unterscheiden (vergleiche EP-A 768 336, den die Seiten 26 und 27 überbrückenden Absatz).

Im Rahmen der Erfindung lassen sich auch solche feinteiligen, wasserunlöslichen Natriumalumosilikate verwenden, die in Gegenwart von wasserlöslichen anorganischen oder organischen Dispergiermitteln gefällt und kristallisiert wurden. Diese können in beliebiger Weise vor oder während der Fällung bzw. Kristallisation in das Reaktionsgemisch eingebracht werden.

Im Rahmen der vorliegenden Erfindung sind Na-Zeolith A und Na-Zeolith P ganz besonders bevorzugt.

Bei den kationischen **Schichtgitterverbindungen b2)** handelt es sich um bekannte Verbindungen, deren Struktur und Herstellung beispielsweise von W. T. Reichle in **Chemtec (Januar 1986), Seiten 58-63,** beschrieben werden.
Der Prototyp kationischer Schichtgitterverbindungen ist das Mineral Hydrotalcit [Mg₆Al₂(OH)₁₆](CO₃) · 4 H₂O. Hydrotalcit leitet sich strukturell vom Brucit [Mg(OH)₂] ab. Brucit kristallisiert in einer Schichtstruktur mit den Metallionen in Oktaederlücken zwischen zwei Schichten aus hexagonal dicht gepackten (OH⁻)-Ionen. Dabei wird nur jede zweite Schicht der Oktaederlücken von Metallionen M besetzt, so daß Schichtpakete (OH)-M-(OH) entstehen. Die Zwischenschichten sind im Brucit leer, im Hydrotalcit sind einige - etwa jede zweite bis fünfte - der Mg(II)-Ionen statistisch durch Al(III)-Ionen ersetzt. Das Schichtpaket erhält dadurch insgesamt eine positive Ladung. Diese Ladung wird durch Anionen ausgeglichen, die sich zusammen mit leicht entfernbarem Kristallwasser in den Zwischenschichten befinden. Schema 1 zeigt - schematisch - den Schichtaufbau von Hydrotalcit.

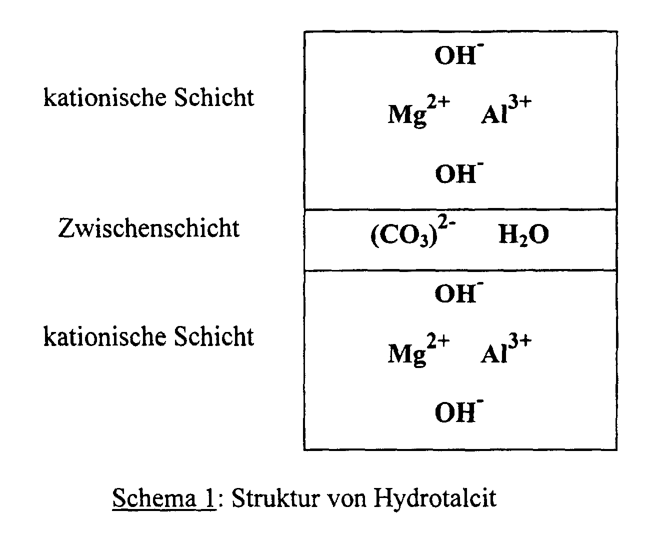

Hydrotalcite bilden pulverige, sich talkig anfühlende Massen mit BET-Oberflächen bis zu etwa 150 m²/g. Zwei Grundsynthesen sind literaturbekannt: Eine Möglichkeit der Synthese besteht darin, wäßrige Lösungen der entsprechenden Metallsalze mit Lauge zu behandeln, wobei der sich bildende Hydrotalcit ausfällt. Eine andere Möglichkeit geht von wasserunlöslichen Ausgangsverbindungen wie Metalloxiden und -hydroxiden aus. Es handelt sich hierbei um heterogene Reaktionen, die üblicherweise im Autoklaven ausgeführt werden.

Wie bereits erwähnt ist Hydrotalcit lediglich der Prototyp kationischer Schichtverbindungen. Die vom Hydrotalcit bekannten Synthesemethoden werden jedoch auch allgemein zur Synthese beliebiger kationischer Schichtverbindungen herangezogen. Wie dem Fachmann bekannt lassen sich diese Synthesemethoden ganz allgemein als Hydrothermalsynthese klassifizieren. Unter Hydrothermalsynthese im engeren Sinne versteht man dabei die Synthese von Mineralien aus hocherhitzten - oberhalb einer Temperatur von 100 °C und einem Druck von 1 atm - wäßrigen Suspensionen; Hydrothermalsynthesen werden meist in Druckgefäßen ausgeführt, da die angewendeten Temperaturen weit über der Siedetemperatur des Wassers liegen, meist sogar über dessen kritischer Temperatur (vergleiche **Römpps Chemie-Lexikon,** ^{**7**}**1973, S. 1539)**

Unter kationischen Schichtgitterverbindungen b2) werden im Rahmen der vorliegenden Erfindung Verbindungen der allgemeinen Formel (III) verstanden

[EₑZ_{z}D_{d}Vᵥ(OH⁻)ₓ](Aⁿ⁻)ₐ^{·}q H₂O (III)

worin bedeuten:
- E ein einwertiges Kation aus der Gruppe der Alkalimetalle,
- e eine Zahl im Bereich von 0 bis 2,
- Z ein zweiwertiges Metall-Kation,
- z eine Zahl im Bereich von 0 bis 6,
- D ein dreiwertiges Metall-Kation,
- d eine Zahl im Bereich von 0 bis 3,
- V ein vierwertiges Metall-Kation,
- v eine Zahl im Bereich von 0 bis 1,
- (Aⁿ⁻) ein Säureanion der Ladung n-, wobei n eine ganze Zahl von 1 bis 3 ist, und
- q eine Zahl im Bereich von 1 bis 10,
- mit der Maßgabe, daß x > a und e + 2z + 3d + 4v = x + na ist.

In einer Ausführungsform hat v in der allgemeinen Formel (III) den Wert Null. Diese Schichtverbindungen lassen sich mithin durch die allgemeine Formel (III*) beschreiben:

[EₑZ_{z}D_{d} (OH⁻)ₓ](Aⁿ⁻)ₐ^{·}q H₂O (III*)

worin bedeuten:
- E ein einwertiges Kation aus der Gruppe der Alkalimetalle,
- e eine Zahl im Bereich von 0 bis 2,
- Z ein zweiwertiges Metall-Kation,
- z eine Zahl im Bereich von 0 bis 6,
- D ein dreiwertiges Metall-Kation,
- d eine Zahl im Bereich von 0 bis 3,
- (Aⁿ⁻) ein Säureanion der Ladung n-, wobei n eine ganze Zahl von 1 bis 3 ist, und
- q eine Zahl im Bereich von 1 bis 10,
- mit der Maßgabe, daß x > a und e + 2z + 3d = x + na ist.

In einer weiteren Ausführungsform hat e in der allgemeinen Formel (III) den Wert Null. Diese Schichtverbindungen lassen sich mithin durch die allgemeine Formel (III**) beschreiben:

[Z_{z}D_{d}Vᵥ(OH⁻)ₓ](Aⁿ⁻)ₐ^{·}q H₂O (III**)

worin bedeuten:
- Z ein zweiwertiges Metall-Kation,
- z eine Zahl im Bereich von 0 bis 6,
- D ein dreiwertiges Metall-Kation,
- d eine Zahl im Bereich von 0 bis 3,
- V ein vierwertiges Metall-Kation,
- v eine Zahl im Bereich von 0 bis 1,
- (Aⁿ⁻) ein Säureanion der Ladung n-, wobei n eine ganze Zahl von 1 bis 3 ist, und
- q eine Zahl im Bereich von 1 bis 10,
- mit der Maßgabe, daß x > a und 2z + 3d + 4v = x + na ist.

In einer bevorzugten Ausführungsform haben e und v in der allgemeinen Formel (III) jeweils den Wert Null. Diese Schichtverbindungen lassen sich mithin durch die allgemeine Formel (III***) beschreiben:

[Z_{z}D_{d}(OH⁻)ₓ](Aⁿ⁻)ₐ^{·}q H₂O (III***)

worin bedeuten:
- Z ein zweiwertiges Metall-Kation,
- z eine Zahl im Bereich von 0 bis 6,
- D ein dreiwertiges Metall-Kation,
- d eine Zahl im Bereich von 0 bis 3,
- (Aⁿ⁻) ein Säureanion der Ladung n-, wobei n eine ganze Zahl von 1 bis 3 ist, und
- q eine Zahl im Bereich von 1 bis 10,
- mit der Maßgabe, daß x > a und 2z + 3d = x + na ist.

Die Schichtverbindungen gemäß Formel (III***) haben hinsichtlich der Zusammensetzung mithin die dem Fachmann bekannte Struktur der "klassischen" Hydrotalcite. Von diesen sind wiederum jene bevorzugt, bei denen D Aluminium, d die Zahl 1 und z eine Zahl im Bereich von 1 bis 5 bedeuten. Diese speziellen Hydrotalcite werden durch die allgemeine Formel (III****) charakterisiert:

[Z_{z}Al(OH⁻)ₓ](Aⁿ⁻)ₐ^{·}q H₂O (III***)

worin bedeuten:
- Z ein zweiwertiges Metall-Kation,
- z eine Zahl im Bereich von 1 bis 5,
- (Aⁿ⁻) ein Säureanion der Ladung n-, wobei n eine ganze Zahl von 1 bis 3 ist, und
- q eine Zahl im Bereich von 1 bis 10,
- mit der Maßgabe, daß x > a und 2z + 3 = x + na ist.

Bevorzugt im Sinne der Erfindung sind solche kationische Schichtverbindungen (III), in der Z für mindestens ein zweiwertiges Metallion, ausgewählt aus der Gruppe Magnesium, Calcium und Zink steht. Bevorzugt steht Z für genau ein zweiwertiges Metallion aus der genannten Gruppe und insbesondere für Magnesium. Ganz besonders bevorzugt werden kationische Schichtverbindungen der allgemeinen Formel I, in denen Aⁿ⁻ für ein Säureanion mit der Ladung (n-) ausgewählt aus der Anionengruppe Carbonat, Hydrogencarbonat, Perchlorat, Acetat, Nitrat, Tartrat, Oxalat und Jodid steht, vorzugsweise für Carbonat. Wenn bei der Erläuterung zu obiger Formel I von mindestens einem zweiwertigen Metallion die Rede ist, so bedeutet dies, daß in der kationischen Schichtverbindung unterschiedliche zweiwertige Metallionen nebeneinander vorliegen können. Die Indices x, y und z sowie m können ganze oder gebrochene Zahlen innerhalb der angegebenen Bedingungen sein. Besonders vorteilhaft sind kationische Schichtverbindungen der allgemeinen Formel (III), in der Z für Magnesium und Aⁿ⁻ für Carbonat steht.

Bei den **CHAP-Verbindungen b3)** handelt es sich um **C**alcium-**H**ydroxy-**A**luminium-(hydrogen)**p**hosphite und/oder deren Hydrate. Diese Verbindungen haben die allgemeine Formel (IV-a)

CaₓAl₂(OH)₂₍ₓ₊₂₎HPO₃·mH₂O (IV-a),

worin:
- x = eine Zahl im Bereich von 2 bis 8 und
- m = eine Zahl im Bereich von 0 bis 12,
bzw. die allgemeine Formel (IV-b)

CaₓAl₂ (OH)_{2(x+3-y)}(HPO₃)_{y}·mH₂O (IV-b),

worin:
- x = eine Zahl im Bereich von 2 bis 12,
- die Bedingung gilt, daß (2x + 5) / 2> y > 0 und
- m = eine Zahl im Bereich von 0 bis 12,
- mit der Maßgabe, daß y = 1 ausgenommen ist, sofern x = eine Zahl im Bereich von 2 bis 8 ist.

Die CHAP-Verbindungen können beispielsweise mittels einem Verfahren hergestellt werden, bei dem man Mischungen aus Calciumhydroxid und/oder Calciumoxid, Aluminiumhydroxid und Natriumhydroxid oder aus Calciumhydroxid und/oder Calciumoxid und Natriumaluminat mit phosphoriger Säure in zur Herstellung der erwünschten Calcium-Aluminium-Hydroxy-Hydrogenphosphite entsprechenden Mengen in wässrigem Medium umsetzt und das Reaktionsprodukt in an sich bekannter Weise abtrennt und gewinnt. Das aus der oben beschriebenen Umsetzung direkt anfallende Reaktionsprodukt kann nach bekannten Verfahren vom wässrigen Reaktionsmedium abgetrennt werden, vorzugsweise durch Filtration. Die Aufarbeitung des abgetrennten Reaktionsproduktes erfolgt ebenfalls in an sich bekannter Weise, beispielsweise durch Waschen des Filterkuchens mit Wasser und Trocknen des gewaschenen Rückstands bei Temperaturen von beispielsweise 60 - 130°C, vorzugsweise bei 90 - 120°C.

Für die Umsetzung kann sowohl feinteiliges, aktives Aluminiumhydroxid in Kombination mit Natriumhydroxid als auch ein Natriumaluminat eingesetzt werden. Calcium kann in Form von feinteiligem Calciumoxid oder Calciumhydroxid oder Mischungen daraus verwendet werden. Die phosphorige Säure kann in unterschiedlicher konzentrierter Form eingesetzt werden. Die Umsetzungstemperaturen liegen vorzugsweise zwischen 50 und 100°C, weiter vorzugsweise zwischen etwa 60 und 85°C. Katalysatoren oder Beschleuniger sind nicht erforderlich, stören aber auch nicht. Bei den Verbindungen kann das Kristallwasser ganz oder teilweise durch thermische Behandlung entfernt werden.

Bei ihrer Anwendung als Stabilisatoren spalten die getrockneten Calcium-Hydroxy-Aluminium-hydroxyphosphite bei den beispielsweise für Hart-PVC üblichen Verarbeitungstemperaturen von 160 - 200°C kein Wasser ab, so dass in den Formteilen keine störende Blasenbildung auftritt.

Zur Verbesserung ihrer Dispergierbarkeit in halogenhaltigen thermoplastischen Harzen können die CHAP-Verbindungen in bekannter Weise mit oberflächenaktiven Mittel beschichtet werden.

Bei den **Katoiten b4**) handelt es sich um Verbindungen der Struktur (V)

Ca₃Al₂(OH)₁₂.mH₂O (V),

wobei m eine Zahl im Bereich von 0 bis 10 bedeutet. Die Katoite können gegebenenfalls oberflächenmodifiziert sein. Sie haben ein ganz bestimmtes Kristallgitter (sogenannte Hydrogranatstruktur), wodurch sie sich von anderen Calcium-Aluminium-Hydroxyverbindungen unterscheiden. Dieses Kristallgitter samt Gitterabständen wird in dem Artikel von C. Cohen-Addad et P. Ducros in Acta Cryst. (1967), 23, Seiten 220 bis 225 beschrieben. Demnach handelt es sich um ein kubisches Kristallgitter. Das Aluminium wird oktaedrisch umgeben von sechs Sauerstoffen, die je noch ein Wasserstoff tragen. Das Calcium ist von 8 Sauerstoffen umgeben, die einen gestörten Kubus bilden, der auch als triangularer Dodekaeder bezeichnet wird.
Die Katoite der allgemeinen Formel Ca₃Al₂(OH)₁₂ können beispielsweise in Anlehnung in die deutsche Patentschrift DE 2 424 763 aus den Hydroxiden des Calciums und Aluminiums in entsprechenden stöchiometrischen Mengen im wässrigen System hergestellt werden. Sie fallen je nach Versuchstemperaturen und Reaktionszeiten mit unterschiedlichen mittleren Teilchendurchmessern an.

Bevorzugt werden Temperaturen im Bereich von 50 bis 150 °C und Reaktionszeiten von 0,1 bis 9 Stunden. Dabei fallen die Katoite mit mittleren Teilchendurchmessern von 0,1 bis 100 µm, vorzugsweise 0,5 bis 30 µm an. Es kann vorkommen, dass als Nebenprodukt geringe Mengen an calciumhaltigen Hydroxyaluminaten (Hydrocalumite) anfallen, die eine Schichtstruktur aufweisen und durch die oben beschriebene allgemeine Formel wiedergegeben werden.
Bei der Herstellung der Katoite können auch Überschüsse von Aluminium- oder Calciumhydroxid eingesetzt werden, wobei Mischungen von nicht umgesetztem Calcium- und/oder Aluminiumhydroxid und Katoit entstehen. Diese Mischungen können ebenfalls im Sinne der Erfindung verwendet werden.
Falls gewünscht, können die Katoite der obigen Formel oberflächenmodifiziert sein mit einem oder mehreren Additiven ausgewählt aus den Gruppen
v-a) gegebenenfalls alkoxylierte Alkohole mit einer oder mehreren Hydroxylgruppen,
v-b) teilweise oder vollständig epoxidierte ungesättigte Fettsäuren, Fettalkohole und/oder deren Derivate,
v-c) Voll- und Partialester von Polyolen mit 3 bis 30 C-Atomen und 2 bis 6 Hydroxylgruppen mit Carbonsäuren mit 6 bis 22 C-Atomen,
v-d) Alkyl- und Arylphosphiten,
v-e) Homo- und Mischpolymeren von Acrylsäure und Methacrylsäure,
v-f) Lignin- und Naphthalinsulfonate und/oder Trimerfettsäuren,
v-g) Salze von Fettsäuren.

Als Additive kommen in der Gruppe v-a) sowohl monofunktionelle Alkohole als auch Polyole mit 3 bis 30 C-Atomen und 2 bis 6 Hydroxylgruppen in Betracht, die gegebenenfalls alkoxyliert, vorzugsweise ethoxyliert sein können. Aus der Gruppe der monofunktionellen Alkohole werden bevorzugt Fettalkohole mit 6 bis 22 C-Atomen eingesetzt wie Caprin-, Lauryl-, Palmityl-, Stearyl- , Oleyl-, Linolyl-, Arachidyl- und Behenylalkohol sowie ihre technischen Mischungen wie sie aus natürlichen Ölen und Fetten zugänglich sind. Ganz besonders bevorzugt werden von diesen Fettalkoholen die ethoxylierten Vertreter hiervon eingesetzt mit 2 bis 15 Mol Ethylenoxid. Aus der Gruppe der Polyole eignen sich Diole mit 3 bis 30 C-Atomen, wie Butandiole, Hexandiole, Dodecandiole, sowie Trimethylolpropan, Pentaerythrit, Glycerin und deren technische Oligomerengemische mit durchschnittlichen Kondensationsgraden von 2 bis 10. Ganz besonders bevorzugt werden aus der Gruppe der Polyole solche mit 3 bis 30 C-Atomen, die pro 3 C-Atomen mindestens eine Hydroxylgruppe oder einen Ethersauerstoff tragen, vorzugsweise Glycerin und/oder die technischen Oligoglyceringemische mit durchschnittlichen Kondenstionsgraden von 2 bis 10.

Bei den Additiven der Gruppe v-b) handelt es sich um teilweise oder vollständig epoxidierte ungesättigte Fettsäuren oder Fettalkohole mit 6 bis 22 C-Atomen oder Derivate hiervon. Als Derivate der epoxidierten Fettsäuren oder Fettalkoholen sind insbesondere die Ester hiervon geeignet, wobei die epoxidierten Fettsäuren und epoxidierten Fettalkohole miteinander verestert sein können oder aber auch mit nichtepoxidierten Carbonsäuren oder mit nichtepoxidierten ein- oder mehrwertigen Alkoholen. Die epoxidierten Fettsäuren leiten sich vorzugsweise von der ungesättigten Palmitolein-, Öl-, Elaidin-, Petroselin-, Ricinol-, Linolen-, Gadolein-, oder Erucasäure ab, die nach bekannten Verfahren ganz oder teilweise epoxidiert werden. Die epoxidierten Fettalkohole leiten sich vorzugsweise ab von den ungesättigten Alkoholen Oleyl-, Elaidyl-, Ricinol-, Linoleyl- , Linolenyl-, Gadoleyl-, Arachidon- oder Erucaalkohol ab, die ebenfalls nach bekannten Verfahren ganz oder teilweise epoxidiert werden. Geeignete Ester von epoxidierten Fettsäuren sind Ester von ein-, zwei- und/oder dreiwertigen Alkoholen, die vollständig mit epoxidierten, ungesättigten Carbonsäuren mit 6 bis 22 C-Atomen verestert sind wie Methyl-, 2-Ethylhexyl-, Ethylenglykol-, Butandiol-, Neopentylglykol-, Glycerin- und/oder Trimethylolpropanester von epoxidierter Lauroleinsäure, Palmitoleinsäure, Ölsäure, Ricinolsäure, Linolsäure und/oder Linolensäure. Bevorzugt werden

Ester von dreiwertigen Alkoholen und praktisch vollständig epoxidierten ungesättigten Carbonsäuren mit 12 bis 22 C-Atomen, und insbesondere Ester von Glycerin mit praktisch vollständig epoxidierten ungesättigten Carbonsäuren mit 12 bis 22 C-Atomen. Wie in der Fettchemie üblich, können die epoxidierten Carbonsäureglyceride auch technische Gemische darstellen, wie man sie durch Epoxidation von natürlichen ungesättigten Fetten und ungesättigten Ölen erhält. Vorzugsweise wird epoxidiertes Rüböl, epoxidiertes Sojaöl und epoxidiertes Sonnenblumenöl neuer Züchtung eingesetzt.

Bei den Additiven der Gruppe v-c) handelt es sich um Voll- oder Partialester, die nach den einschlägigen Methoden der präparativen organischen Chemie, beispielsweise durch säurekatalysierte Umsetzung von Polyolen mit Carbonsäuren erhalten werden. Als Polyolkomponente kommen dabei solche in Betracht, die bereits in Zusammenhang mit der Gruppe a) besprochen wurden. Als Säurekomponente werden bevorzugt aliphatische, gesättigte und/oder ungesättigte Carbonsäuren mit 6 bis 22 C-Atomen wie Capronsäure, Caprylsäure, Caprinsäure, Laurinsäure, Myrisitinsäure, Palmitinsäure, Palmitoleinsäure, Stearinsäure, Ölsäure, Ricinolsäure, Linolsäure, Linolensäure, Behensäure oder Erucasäure eingesetzt. Wie in der Fettchemie üblich, kann die Carbonsäure auch ein technisches Gemisch darstellen, wie es bei der Druckspaltung von natürlichen Fetten und Ölen anfällt. Bevorzugt werden Partialester von Glycerin und insbesondere von deren technischen Oligoglyceringemischen mit durchschnittlichen Kondensationsgraden von 2 bis 10 mit gesättigten und/oder ungesättigten aliphatischen Carbonsäuren mit 6 bis 22 C- Atomen.

Schliesslich können gemäss Gruppe v-d) Alkyl- und Arylphosphite eingesetzt werden, vorzugsweise solche der allgemeinen Formel (V) 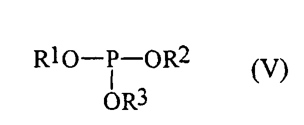 in der R¹, R² und R³ unabhängig voneinander für einen Alkylrest mit 1 bis 18 Kohlenstoffatomen oder einen Phenylrest stehen. Typische Beispiele für Additive der Gruppe d) sind Tributylphosphit, Triphenylphosphit, Dimethylphenylphosphit und/oder Dimethylstearylphosphit. Bevorzugt wird Diphenyldecylphosphit.

Bei den Additiven aus der Gruppe v-e) handelt es sich bevorzugt um Polymere von Acrylsäure und Methacrylsäure sowie deren Copolymeren. Der Begriff der Copolymere wird in doppeltem Sinne verstanden: einmal als reine Copolymere von Acrylsäure und Methacrylsäure und zum anderen als Copolymere von (Meth)Acrylsäure mit weiteren vinylisch ungesättigten, zur Polymerisation fähigen Monomeren. Beispiele für weitere zur Polymerisation fähige Monomere sind sulfonund phosphonsäuregruppenhaltige ungesättigte Monomere, ungesättigte aliphatische Carbonsäuren mit 3 bis 5 C-Atomen, Amide von ungesättigten aliphatischen Carbonsäuren mit 3 bis 5 C-Atomen, aminogruppenhaltige ungesättigte Monomere und/oder deren Salze, Vinylacetat, Acrolein, Vinylchlorid, Acrylnitril, Vinylidenchlorid, 1,3-Butadien, Styrol, Alkylstyrole mit 1 bis 4 C-Atomen im Alkylrest. Beispiele für Additive der Gruppe v-e) sind Polyacrylsäure, Polymethacrylsäure - im folgenden werden Acrylsäure und Methacrylsäure sowie deren Derivate vereinfacht als (Meth)acrylsäure bzw. Derivate abgekürzt - und/oder deren Salze wie Polynatrium(meth)acrylat, Copolymere von (Meth)acrylsäure mit Maleinsäure, Maleinsäureanhydrid, Styrolsulfonsäure, α-Methylstyrol, 2-Vinylpyridin, 1-Vinylimidazol, Dimethylaminopropyl(meth)acrylamid, 2-(meth)acrylamido-2-methylpropansulfonsäure, (Meth)acrylamid, N-Hydroxydimethyl(meth)acrylamid und/oder deren Salze. Ganz besonders bevorzugt unter den polymeren Additiven sind solche, die einen überwiegend anionischen Charakter aufweisen, das heisst, die mehrheitlich Säuregruppen frei oder in Form ihrer Salze tragen. Insbesondere bevorzugt sind Polymere von (Meth)acrylsäure sowie deren Copolymerisate mit Styrol, Acrolein, Alkylstyrolen mit 1 bis 4 C-Atomen im Alkylrest, Styrolsulfonsäure, Maleinsäure und/oder deren Salze, insbesondere deren Natriumsalze und Maleinsäureanhydrid. Zweckmässigerweise besitzen die polymeren Additive der Gruppe e) ein Molekulargewicht von 1000 bis 10000. Die Herstellung der polymeren Additive kann nach bekannten Verfahren wie Substanz- oder Lösungsmittelpolymerisation erfolgen (vergleiche dazu Ullmann's Encyclopädie der technischen Chemie, Band 19, 4. Auflage, Seiten 2 - 11, 1980).

Die den Additiven der Gruppe v-g) handelt es sich um Salze von Fettsäuren. Geeignete Fettsäuren wurden bereits im Zusammenhang mit Additiven der Gruppe v-c) aufgezählt. Bevorzugt werden hier die Alkalisalze der gesättigten Fettsäuren.

Ein oder mehrere Additive aus einer oder mehreren der Gruppen v-a) bis v-g) können zur Modifizierung der Katoite eingesetzt werden, wobei die Gesamtmenge an Additiven in dem Bereich von 0, 1 bis 10 Gew% - bezogen auf Katoit - liegt. Bei Kombinationen der polymeren Additive v-e) mit weiteren Additiven aus den Gruppen v-a) bis v-d) und v-f) und v-g) ist es bevorzugt, die Additive in Mengen von 50 bis 90 Gew% - bezogen auf die Gesamtadditivmenge - zu haben. Besonders bevorzugt werden von den oberflächenmodifizierten Katoiten solche, die mit einem oder mehreren Additiven aus den Gruppen v-b), v-e) und v-g) modifiziert sind.

Die Modifizierung der Katoite kann entweder in situ oder nachträglich erfolgen.

Bei der nachträglichen Modifzierung werden die Katoite mit organischen oder wässrigen Lösungen der Additive innigst vermahlen, vorzugsweise mit Mahlkörpermühlen und insbesondere mit einer Kugelmühle und anschliessend gewöhnlich getrocknet. Sofern es sich bei den Additiven um bei Raumtemperatur flüssige oder niedrigschmelzende Produkte handelt, muss man natürlich keine Lösungen davon verwenden. Ansonsten verwendet man bei den Additiven v-a) bis v-g) am liebsten klare wässrige Lösungen oder Lösungen mit polaren organischen Lösungsmitteln.

Der Begriff der polaren organischen Lösungsmittel umfasst bei Raumtemperatur (15 bis 25°C) flüssige Kohlenwasserstoffverbindungen, die mindestens eine elektronegativeren Substituenten als Kohlenstoff tragen. Dazu zählen Chlorkohlenwasserstoffe, Alkohole, Ketone, Ester, Ether und/oder Glykolether. Geeignete polare organische Lösungsmittel sind Methanol, Ethanol, n-Butanol, Aceton, Methylethylketon, Methylisobutylketon, Cyclohexanol, Isophoron, Ethylacetat, Milchsäureethylester, 2-Methoxyethylacetat, Tetrahydrofuran, Ethylglykolmonomethylether, Diethylenglykolmonoethylether.

Damit die Oberfläche der Katoite gleichmässig modifiziert werden kann, ist es bei Anwesenheit der Additive der Gruppe v-e) zweckmässig, wenn diese löslich sind in polaren organischen Lösungsmitteln der beschriebenen Art und/oder Wasser mit pH-Werten von 8 bis 12. Der Begriff löslich bedeutet in diesem Zusammenhang, dass die polymeren Additive v-e) in den polaren organischen Lösungsmitteln und in einer wässrigen Lösung mit pH 10, eingestellt mit Alkalihydroxiden bei 20°C, zu mindestens 0,01 Gew.-%, vorzugsweise 0,1 Gew.-% - bezogen auf die Lösung - und insbesondere unter den angegebenen Bedingungen vollständig klar gelöst sind.

Die Modifizierung kann auch in situ erfolgen, das heisst, man kann bereits den Calcium- und Aluminiumhydroxid-Lösungen, aus dem sich der Katoit bildet, die Additive gegebenenfalls in Form ihrer Lösungen zusetzen.

Letztendlich kann man aber auch beide Modifizierungsarten kombinieren, was sich für die Modifizierung mit mehreren Additiven empfiehlt, die insbesondere unterschiedliches Lösungsverhalten zeigen.

Die Verbindungen b) können in der Matrix in einer Menge von 1 bis 80 Gew.-Teilen, bezogen auf 100 Gew.-Teile der Matrix, enthalten sei. Mengen von 5 bis 50 Gew.-Teilen sind dabei bevorzugt.

In einer Ausführungsform enthält die Matrix **halogenhaltige organische Kunststoffe c).** Bei halogenhaltigen organischen Kunststoffen, handelt es sich insbesondere um chlorhaltige Polymere oder deren Recyclate. Beispiele für solche zu stabilisierenden chlorhaltigen Polymere oder deren Recyclate sind: Polymere des Vinylchlorides, Vinylharze, enthaltend Vinylchlorideinheiten in deren Struktur, wie Copolymere des Vinylchlorids und Vinylester von aliphatischen Säuren, insbesondere Vinylacetat, Copolymere des Vinylchlorids mit Estern der Acryl- und Methacrylsäure und mit Acrylnitril, Copolymere des Vinylchlorids mit Dienverbindungen und ungesättigten Dicarbonsäuren oder deren Anhydride, wie Copolymere des Vinylchlorids mit Diethylmaleat, Diethylfumarat oder Maleinsäureanhydrid, nachchlorierte Polymere und Copolymere des Vinylchlorids, Copolymere des Vinylchlorids und Vinylidenchlorids mit ungesättigten Aldehyden, Ketonen und anderen, wie Acrolein, Crotonaldehyd, Vinylmethylketon, Vinylmethylether, Vinylisobutylether und ähnliche; Polymere des Vinylidenchlorids und Copolymere desselben mit Vinylchlorid und anderen polymerisierbaren Verbindungen; Polymere des Vinylchloracetates und Dichlordivinylethers; chlorierte Polymere des Vinylacetates, chlorierte polymerische Ester der Acrylsäure und der alpha-substituierten Acrylsäure; Polymere von chlorierten Styrolen, zum Beispiel Dichlorstyrol; chlorierte Polymere des Ethylens; Polymere und nachchlorierte Polymere von Chlorbutadiens und deren Copolymere mit Vinylchlorid; sowie Mischungen der genannten Polymere unter sich oder mit anderen polymerisierbaren Verbindungen.

Ferner sind umfaßt die Pfropfpolymerisate von PVC mit EVA, ABS und MBS. Bevorzugte Substrate sind auch Mischungen der vorstehend genannten Homound Copolymerisate, insbesondere Vinylchlorid-Homopolymerisate, mit anderen thermoplastischen oder/und elastomeren Polymeren, insbesondere Blends mit ABS, MBS, NBR, SAN, EVA, CPE, MBAS, PMA, PMMA, EPDM und Polylactonen.

Weiterhin bevorzugt sind Suspensions- und Massepolymere, sowie Emulsionspolymere.

Als chlorhaltiges Polymerisat ist Polyvinylchlorid besonders bevorzugt, insbesondere Suspensionspolymerisat und Massepolymerisat.

Im Rahmen dieser Erfindung sind unter PVC auch Copolymerisate oder Pfropfpolymerisate von PVC mit polymerisierbaren Verbindungen wie Acrylnitril, Vinylacetat oder ABS zu verstehen, wobei es sich um Suspensions-, Masse- oder Emulsionspolymerisate handeln kann. Bevorzugt ist PVC Homopolymer auch in Kombination mit Polyacrylaten.

Weiterhin kommen als Verbindungen c) im Rahmen dieser Erfindung auch Recyclate chlorhaltiger Polymere in Frage, wobei es sich hierbei um die oben näher beschriebenen Polymere handelt, welche durch Verarbeitung, Gebrauch oder Lagerung eine Schädigung erfahren haben. Besonders bevorzugt ist PVC-Recyclat. In den Recyclaten können auch kleine Mengen an Fremdstoffen enthalten sein, wie z.B. Papier, Pigmente, Klebstoffe, die oft schwierig zu entfernen sind. Diese Fremdstoffe können auch aus dem Kontakt mit diversen Stoffen während des Gebrauchs oder der Aufarbeitung stammen, wie z.B. Treibstoffreste, Lackanteile, Metallspuren und Initiatorreste.

In einer Ausführungsform enthält die Matrix ein oder mehrere Additive, die ausgewählt sind aus der Gruppe der
(d1) Glycidyl-Verbindungen,
(d2) beta-Diketone und beta-Ketoester,
(d3) Dihydropyridine und Polydihydropyridine,
(d4) Polyole und Disaccharidalkohole,
(d5) sterisch gehinderte Amine (Tetraalkylpiperidinverbindungen),
(d6) Alkalialumocarbonate (Dawsonite),
(d7) Alkali- und Erdalkalihydroxide, -hydrogencarbonate und -carbonate,
(d8) Antioxidantien,
(d9) Trenn - und/oder Gleitmittel,
(d10) Weichmacher,
(d11) Pigmente,
(d12) Füllstoffe,
(d13) Phosphite,
(d14) Thiophosphite und Thiophosphate,
(d15) Mercaptocarbonsäureester,
(d16) Epoxidierte Fettsäureester,
(d17) UV-Absorber und Lichtschutzmittel,
(d18) Treibmittel,
(d19) Harnstoff.

Die Verbindungen der Klassen (d1) bis (d19) sind dem Fachmann als Additive für halogenhaltige Kunststoffe, insbesondere PVC, wohlbekannt. Für repräsentative Beispiele von Substanzen aus diesen Klassen sei beispielhaft auf die eingangs zitierte EP-A-768 336 verwiesen.

Die Additive (d1) bis (d19) können wie folgt als Matrix-Komponenten eingesetzt werden:
- einzeln,
- im Gemisch untereinander,
- in Kombination mit ein oder mehreren der möglichen Matrix-Komponenten a), b) und c).

In Zusammenhang mit dem Gebrauch des Begriffs **"Additiv"** sei jedoch darauf hingewiesen, daß der Fachmann auf dem Gebiet der Kunststoffverarbeitung die Additive sowohl unter strukturellen als auch unter funktionellen Gesichtspunkten klassifiziert.

Bei funktioneller Klassifizierung sind typische Kunststoffadditive: Antistatika, Antischleiermittel, Antioxidantien, UV-Stabilisatoren, Haftmittel, Kalandrierhilfen, Formtrennmittel, Gleitmittel, Trennmittel, Schmiermittel, Weichmacher, Duftmittel, Flammschutzmittel, Füllstoffe, Pigmente, Treibmittel, Mittel zur Erhöhung der Thermostabilität (Thermostabilisatoren).

Die oben genannten Additivklassen (d1) bis (d19) folgen weitgehend der strukturellen Klassifizierung, d.h. der Klassifizierung hinsichtlich der chemischen Struktur. Bei den Klassen (d8) bis (d12) sowie (d17) und (d18) wurde jedoch die funktionelle Definition vorgezogen.

Es sei weiterhin darauf hingewiesen, daß Verbindungen einer bestimmten Stoffklasse, mithin Verbindungen die unter strukturellen Gesichtspunkten derselben Klasse zugeordnet werden können, in der Praxis häufig nicht nur eine Funktion erfüllen, sondern zwei oder mehrere. So können beispielsweise Calciumseifen als Gleitund/oder Trennmittel wirken, sie können aber auch - etwa bei der Verarbeitung des Kunststoffes Polyvinylchlorid (PVC) - als Mittel zur Verbesserung der Thermostabilität dienen.

### Zu den Substanzen der Gruppen d1) bis d19)

### Glycidylverbindungen d1)

Sie enthalten die Glycidylgruppe 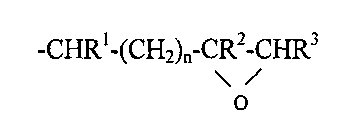 wobei diese direkt an Kohlenstoff-, Sauerstoff-, Stickstoff- oder Schwefelatome gebunden ist, und worin entweder R¹ und R³ beide Wasserstoff sind, R² Wasserstoff oder Methyl und n=0 ist, oder worin R¹ und R³ zusammen -CH₂-CH₂- oder -CH₂-CH₂-CH₂- bedeuten, R² dann Wasserstoff und n=0 oder 1 ist.

Beispiele für geignete Glycidylverbindungen sind solche der nachfolgend beschriebenen Gruppen dl-I) bis dl-V).

### Verbindungen der Gruppe dl-I)

Glycidyl- und β-Methylglycidylester erhältlich durch Umsetzung einer Verbindung mit mindestens einer Carboxylgruppe im Molekül und Epichlorhydrin bzw. Glycerindichlorhydrin bzw. b-Methyl-epichlorhydrin. Die Umsetzung erfolgt zweckmäßig in Gegenwart von Basen.

Als Verbindungen mit mindestens einer Carboxylgruppe im Molekül können aliphatische Carbonsäuren verwandt werden. Beispiele für diese Carbonsäuren sind Glutarsäure, Adipinsäure, Pimelinsäure, Korksäure, Azelainsäure, Sebazinsäure oder dimerisierte bzw. trimerisierte Linolsäure, Acryl- und Methacrylsäure, Capron-, Capryl-, Laurin- , Myristin-, Palmitin-, Stearin- und Pelargonsäure.

Es können aber auch cycloaliphatische Carbonsäuren eingesetzt werden, wie beispielsweise Cyclohexancarbonsäure, Tetrahydrophthalsäure, 4-Methyltetrahydrophthalsäure, Hexahydrophthalsäure oder 4-Methylhexahydrophthalsäure.

Weiterhin können aromatische Carbonsäuren Verwendung finden, wie beispielsweise Benzoesäure, Phthalsäure, Isophthalsäure, Trimellithsäure oder Pyromellithsäure.

Es können auch carboxylterminierte Addukte, z.B. von Trimellithsäure und Polyolen, wie beispielsweise Glycerin oder 2,2-Bis-(4-hydroxycyclohexyl)propan verwandt werden.

### Verbindungen der Gruppe d1-II)

Glycidyl- oder (β-Methylglycidyl)-ether erhältlich durch Umsetzung einer Verbindung mit mindestens einer freien alkoholischen Hydroxygruppe und/oder phenolischen Hydroxygruppe und einem geeignet substituierten Epichlorhydrin unter alkalischen Bedingungen, oder in Anwesenheit eines sauren Katalysators und anschließender Alkalibehandlung.

Ether dieses Typs leiten sich beispielsweise ab von acyclischen Alkoholen, wie Ethylenglykol, Diethylenglykol und höheren Poly-(oxyethylen)-glykolen, Propan-1,2-diol, oder Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Bistrimethylolpropan, Pentaerythrit, Sorbit, sowie von Polyepichlorhydrinen, Butanol, Amylalkohol, Pentanol, sowie von monofünktionellen Alkoholen wie Isooctanol, 2-Ethylhexanol, Isodecanol sowie C₇-C₉-Alkanol- und C₉-C₁₁-Alkanolgemischen.

Sie leiten sich aber auch beispielsweise ab von cycloaliphatischen Alkoholen wie 1,3-oder 1,4-Dihydroxycyclohexan, Bis-(4-hydroxycyclohexyl)-methan, 2,2-Bis-(4-hydroxycyclohexyl)-propan oder 1,1-Bis-(hydroxymethyl)-cyclohex-3-en oder sie besitzen aromatische Kerne wie N,N-Bis-(2-hydroxyethyl)anilin oder p,p'-Bis-(2-hydroxyethylamino)-diphenylmethan.

Die Epoxidverbindungen können sich auch von einkernigen Phenolen ableiten, wie beispielsweise von Phenol, Resorcin oder Hydrochinon; oder sie basieren auf mehrkernigen Phenolen wie beispielsweise auf Bis-(4-hydroxyphenyl)methan, 2,2-Bis-(4-hydroxyphenyl)-propan, 2,2-Bis-(3,5-dibrom-4-hydroxyphenyl)-propan, 4,4'-Dihydroxydiphenylsulfon oder auf unter sauren Bedingungen erhaltene Kondensationsprodukte von Phenolen mit Formaldehyd wie Phenol-Novolake.

Weitere mögliche endständige Epoxide sind beispielsweise: Glycidyl-1-naphthylether, Glycidyl-2-phenylphenylether, 2-Biphenylglycidylether, N-(2,3-epoxypropyl)-phthalimid und 2,3-Epoxypropyl-4-methoxyphenylether.

### Verbindungen der Gruppe d1-III)

(N-Glycidyl)-Verbindungen erhältlich durch Dehydrochlorierung der Reaktionsprodukte von Epichlorhydrin mit Aminen, die mindestens ein Aminowasserstoffatom enthalten. Bei diesen Aminen handelt es sich zum Beispiel um Anilin, N-Methylanilin, Toluidin, n-Butylamin, Bis-(4-aminophenyl)-methan, m-Xylylendiamin oder Bis-(4-methylaminophenyl)-methan, aber auch N,N,O-Triglycidylm-aminophenol oder N,N,O-Triglycidyl-p-aminophenol.

Zu den (N-Glycidyl)-Verbindungen zählen aber auch N,N'-Di-, N,N',N"-Tri- und N,N',N",N'''-Tetraglycidylderivate von Cycloalkylenharnstoffen, wie Ethylenharnstoff oder 1,3-Propylenharnstoff, und N,N'-Diglycidylderivate von Hydantoinen, wie von 5,5-Dimethylhydantoin oder Glykoluril und Triglycidylisocyanurat.

### Verbindungen der Gruppe d1-IV)

S-Glycidyl-Verbindungen, wie beispielsweise Di-S-glycidylderivate, die sich von Dithiolen, wie beispielsweise Ethan-1,2-dithiol oder Bis-(4-mercaptomethylphenyl)-ether ableiten.

### Verbindungen der Gruppe d1-V)

Epoxidverbindungen mit einem Rest der Formel I, worin R₁ und R₃ zusammen -CH₂-CH₂- bedeuten und n=0 ist, sind Bis-(2,3-epoxycyclopentyl)-ether, 2,3-Epoxycyclopentylglycidylether oder 1,2-Bis-(2,3-epoxycyclopentyloxy)-ethan. Ein Epoxidharz mit einem Rest der Formel I, worin R₁ und R₃ zusammen -CH₂-CH₂- sind und n=1 bedeutet, ist beispielsweise 3,4-Epoxy-6-methyl-cyclohexancarbonsäure-(3',4'-epoxy-6'-methyl-cyclohexyl)-methylester.

Geeignete endständige Epoxide sind beispielsweise (™ bedeutet ®):
- flüssige Bisphenol-A-diglycidylether wie Araldit™GY 240, Araldit™GY 250, Araldit™GY 260, Araldit™GY 266, Araldit ™GY 2600, Araldit™MY 790:
- feste Bisphenol- A-diglycidylether wie Araldit™GT 6071, Araldit™GT 7071, Araldit™GT 7072, Araldit™GT 6063, Araldit™GT 7203, Araldit™GT 6064, Araldit™GT 7304, Araldit™GT 7004, Araldit™GT 6084, Araldit™GT1999, Araldit™GT 7077, Araldit™GT 6097, Araldit™GT 7097, Araldit™GT 7008, Araldit™GT 6099, Araldit™GT 6608, Araldit™GT 6609, Araldit™GT 6610;
- flüssige Bisphenol-F-diglycidylether wie Araldit™GY 281, Araldit™PY 302, Araldit ™PY 306:
- feste Polyglycidylether von Tetraphenylethan wie CG Epoxy Resin™0163:
- feste und flüssige Polyglycidylether von Phenolformaldehyd Novolak wie EPN 1138, EPN 1139, GY 1180, PY 307;
- feste und flüssige Polyglycidylether von o-Cresolformaldehyd Novolak wie ECN 1235, ECN 1273, ECN 1280, ECN 1299;
- flüssige Glycidylether von Alkoholen wie Shell™ Glycidylether 162, Araldit™DY 0390, Araldit™DY 0391;
- flüssige Glycidylether von Carbonsäuren wie Shell™Cardura E Terephthalsäureester, Tri mellithsäureester, Araldit™PY 284;
- feste heterocyclische Epoxidharze (Triglycidylisocyanurat) wie Araldit™PT 810;
- flüssige cycloaliphatische Epoxidharze wie Araldit™CY 179;
- flüssige N,N,O-Triglycidylether von p-Aminophenol wie Araldit™MY 0510;
- Tetraglycidyl-4-4'-methylenbenzamin oder N,N,N',N'-Tetraglycidyldiaminophenylmethan wie Araldit™ MY 720, Araldit™MY 721.

Vorzugsweise finden Epoxidverbindungen mit zwei funktionellen Gruppen Verwendung. Es können aber auch Epoxidverbindungen mit einer, drei oder mehr funktionellen Gruppen eingesetzt werden.

Vorwiegend werden Epoxidverbindungen, vor allem Diglycidylverbindungen, mit aromatischen Gruppen eingesetzt.

Gegebenenfalls kann auch ein Gemisch verschiedener Epoxidverbindungen eingesetzt werden.

Besonders bevorzugt sind als endständige Epoxidverbindungen Diglycidylether auf der Basis von Bisphenolen, wie beispielsweise von 2,2-Bis-(4-hydroxyphenyl)-propan (Bisphenol A), Bis-(4-hydroxyphenyl)-methan oder Mischungen von Bis-(ortho/parahydroxyphenyl)-methan (Bisphenol F).

Die endständigen Epoxidverbindungen können in der Matrix in einer Menge von vorzugsweise mindestens 0,1 Teil, beispielsweise 0,1 bis 50, zweckmäßig 1 bis 30 und insbesondere 1 bis 25 Gew.-Teilen, bezogen auf 100 Gew.-Teile der Matrix, eingesetzt werden.

### beta-Diketone und beta-Ketoester d2)

Verwendbare 1,3-Dicarbonylverbindungen können lineare oder cyclische Dicarbonylverbindungen sein. Bevorzugt werden Dicarbonylverbindungen der folgenden Formel eingesetzt

R¹-CO-CHR²-CO-R³,

worin bedeuten:
- R¹: C₁-C₂₂-Alkyl, C₅-C₁₀-Hydroxyalkyl, C₂-C₁₈-Alkenyl, Phenyl, durch OH, C₁-C₄-Alkyl, C₁-C₄-Alkoxy oder Halogen substituiertes Phenyl, C₇-C₁₀-Phenylalkyl, C₅-C₁₂-cycloalkyl, durch C₁-C₄-Alkyl substituiertes C₅-C₁₂-Cycloalkyl oder eine Gruppe -R⁵-S-R⁶ oder -R⁵-O- R⁶,
- R²: Wasserstoff, C₁-C₈-Alkyl, C₂-C₁₂-Alkenyl, Phenyl, C₇-C₁₂-Alkylphenyl, C₇-C₁₀-Phenylalkyl oder eine Gruppe -CO- R⁴,
- R³: eine der für R¹ gegebenen Bedeutungen oder C₁-C₁₈-Alkoxy,
- R⁴: C₁-C₄-Alkyl oder Phenyl,
- R⁵: C₁-C₁₀-Alkylen,
- R⁶: C₁-C₁₂-Alkyl, Phenyl, C₇-C₁₈-Alkylphenyl oder C₇-C₁₀ -Phenylalkyl.

Hierzu gehören die Hydroxylgruppen enthaltenden Diketone der EP-346 279 und die Oxa- und Thia-diketone der EP-307 358 ebenso wie die auf Isocyansäure basierenden Ketoester der US 4,339,383.

R¹ und R³ als Alkyl können insbesondere C₁-C₁₈-Alkyl sein, wie z.B. Methyl, Ethyl, n-Propyl, Isopropyl, n-Butyl, tert Butyl, Pentyl, Hexyl, Heptyl, Octyl, Decyl, Dodecyl oder Octadecyl.

R¹ und R³ als Hydroxyalkyl stellen insbesondere eine Gruppe -(CH₂)ₙ-OH dar, worin n 5, 6 oder 7 ist.

R¹ und R³ als Alkenyl können beispielsweise Vinyl, Allyl, Methallyl, 1-Butenyl, 1-Hexenyl oder Oleyl bedeuten, vorzugsweise Allyl.

R¹ und R³ als durch OH, Alkyl, Alkoxy oder Halogen substituiertes Phenyl können beispielsweise Tolyl, Xylyl, tertButylphenyl, Methoxyphenyl, Ethoxyphenyl, Hydroxyphenyl, Chlorphenyl oder Dichlorphenyl sein.

R¹ und R³ als Phenylalkyl sind insbesondere Benzyl.

R² und R³ als Cycloalkyl oder Alkyl-cycloalkyl sind insbesondere Cyclohexyl oder Methylcyclohexyl.

R² als Alkyl kann insbesondere C₁-C₄-Alkyl sein. R² als C₂-C₁₂-Alkenyl kann insbesondere Allyl sein. R² als Alkylphenyl kann insbesondere Tolyl sein. R² als Phenylalkyl kann insbesondere Benzyl sein. Vorzugsweise ist R² Wasserstoff. R³ als Alkoxy kann z.B. Methoxy, Ethoxy, Butoxy, Hexyloxy, Octyloxy, Dodecyloxy, Tridecyloxy, Tetradecyloxy oder Octadecyloxy sein. R⁵ als C₁-C₁₀-Alkylen ist insbesondere C₂-C₄-Alkylen. R⁶ als Alkyl ist insbesondere C₄-C₁₂-Alkyl, wie z.B. Butyl, Hexyl, Octyl, Decyl oder Dodecyl. R⁶ als Alkylphenyl ist insbesondere Tolyl. R⁶ als Phenylalkyl ist insbesondere Benzyl.

Beispiele für 1,3-Dicarbonylverbindungen der obigen Formel sind Acetylaceton, Butanoylaceton, Heptanoylaceton, Stearoylaceton, Palmitoylaceton, Lauroylaceton, Benzoylaceton, Dibenzoylmethan, Lauroylbenzoylmethan, Palmitoylbenzoylmethan, Stearoyl-benzoylmethan, Isooctylbenzoylmethan, 5-Hydroxycapronyl-benzoylmethan, Tribenzoylmethan, Bis(4-methylbenzoyl)methan, Benzoyl-p-chlorbenzoylmethan, Bis(2-hydroxybenzoyl)methan, 4-Methoxybenzoyl- benzoylmethan, Bis(4-methoxybenzoyl)methan, 1-Benzoyl-1-acetylnonan, Benzoyl-acetyl-phenylmethan, Stearoyl-4-methoxybenzoylmethan, Bis(4-tert-butylbenzoyl)methan, Benzoylformylmethan, Benzoyl-phenylacetylmethan, Bis-cyclohexanoyl-methan, Dipivaloyl-methan, 2-Acetylcyclopentanon, 2-Benzoylcyclo-pentanon, Diacetessigsäuremethyl-, -ethyl- und - allylester, Benzoyl-, Propionyl- und Butyrylacetessigsäuremethyl- und -ethylester, Triacetylmethan, Acetessigsäuremethyl-, -ethyl-, -hexyl-, -octyl-, -dodecyl- oder -octadecylester, Benzoylessigsäuremethyl-, -ethyl-, -butyl-, -2-ethylhexyl-, -dodecyl- oder - octadecylester, sowie Propionyl- und Butyrylessigsäure-C₁-C₁₈- alkylester. Stearoylessigsäureethyl-, -propyl-, -butyl-, -hexyl- oder -octylester sowie mehrkernige β-Ketoester wie in EP 433.230 beschrieben und Dehydracetsäure sowie deren Zink-, Magnesium- oder Alkalisalze.

Bevorzugt sind 1,3-Diketoverbindungen der obigen Formel, worin R¹ C₁-C₁₈-Alkyl, Phenyl, durch OH, Methyl oder Methoxy substituiertes Phenyl, C₇-C₁₀-Phenylalkyl oder Cyclohexyl ist, R² Wasserstoff ist und R³ eine der für R¹ gegebenen Bedeutungen hat.

Die 1,3-Dicarbonylverbindungen der obigen Formel können alleine, als Mischungen und/oder als deren Alkali-, Erdalkali- und Zinkchelate eingesetzt werden.

Die 1,3-Diketoverbindungen können in der Matrix in einer Menge von beispielsweise 0,01 bis 30, zweckmäßig 0,1 bis 20 und insbesondere 2 bis 10 Gew.-Teilen, bezogen auf 100 Gew.-Teile der Matrix, eingesetzt werden.

### Dihydropyridine und Polydihydropyridine d3)

Als monomere Dihydropyridine kommen Verbindungen, wie z. B. in FR 2 039 496, EP 2 007 , EP 362 012 und EP 24 754 beschrieben, in Frage. Bevorzugt sind solche der Formel, 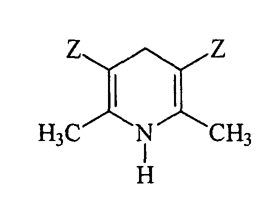 worin Z für CO₂CH₃, CO₂C₂H₅, CO₂ⁿC₁₂H₂₅ oder -CO₂C₂H₄-S- ⁿC₁₂H₂₅ steht. Das hochgestellte n hat dabei die Bedeutung, daß der C₁₂H₂₅-Alkylrest unverzweit ist.

Als Polydihydropyridine kommen vor allem Verbindungen der folgenden Formel in Frage

T―X―R―X―R'―X―L

worin
- X den Rest 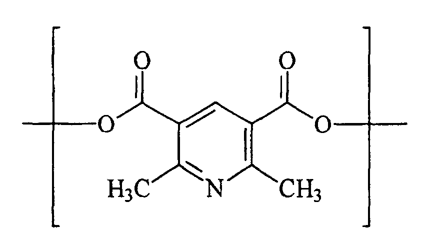 bedeutet,
- T für unsubstituiertes C₁₋₁₂Alkyl steht,
- L dieselben Bedeutungen wie T hat.
- m und n Zahlen von 0 bis 20 bedeuten,
- k die Zahl 0 oder 1 ist,
- R und R' unabhängig voneinander Ethylen, Propylen, Butylen oder eine Alkylenoder Cycloalkylenbismethylengruppe des Typs - (-CₚH₂ₚ-X-)ₜCₚH₂ₚ- sind,
- p eine Zahl im Bereich von 2 bis 8 ist,
- t eine Zahl im Bereich von 0 bis 10 ist
- X fiir Sauerstoff oder Schwefel steht.

Derartige Verbindungen sind in EP 286 887 näher beschrieben.

Die (Poly-)Dihydropyridine können in der Matrix zweckmäßig zu 0,1 bis 30 und insbesondere 1 bis 20 Gew.-Teilen, bezogen auf 100 Gew.-Teile der Matrix, eingesetzt werden.

Besonders bevorzugt sind Thiodiethylen-bis-[5-methoxycarbonyl-2,6-dimethyl-1,4-dihydro pyridin-3-carboxylat] und Thiodiethylen-bis-[5-methoxycarbonyl-2,6-dimethyl-1,4-dihydropyridin- 3-carboxylat].

### Polyole und Polyolerivate d4)

Als Polyole besonders geeignet sind beispielsweise Pentaerythrit, Dipentaerythrit, Tripentaerythrit, Bistrimethylolpropan, Inosit, Polyvinylalkohol, Bistrimethylolethan, Trismethylolpropan, Sorbit, Maltit, Isomaltit, Lactit, Lycasin, Mannit, Lactose, Leucrose, Tris-(hydroxylethyl)isocyanurat (THEIC), Palatinit, Tetramethylolcyclohexanol, Tetramethylolcyclopentanol, Tetramethylolcyclopyranol, Glycerin, Diglycerin, Polyglycerin oder Thiodiglycerin sowie Umsetzungsprodukte dieser Polyole mit Ethylenoxid und/oder Propylenoxid.
Die Polyole können gewünschtenfalls an einer oder an mehreren OH-Gruppen verestert oder verethert sein. Bevorzugt sind solche Polyolderivatae, die Ester von Polyolen mit Carbonsäuren darstellen, etwa Glycerinpartialester von Fettsäuren, beispielsweise Glycerinmonooleat, Glycerindioleat, Glycerinmonostearat, Glycerindistearat, Pentaerytrit- oder TMP-Partialester oder Ester von Dicarbonsäuren (z.B. Adipinsäure, Maleinsäure) mit Polyolen wie Pentaerythrit, Glycerin oder Trismethylolpropan.
Die Polyole bzw. Polyolderivate können allein oder in Mischung untereinander eingesetzt werden.
Die Polyole bzw. Polyolderivate können in der Matrix in einer Menge von beispielsweise 0,01 bis 40, zweckmäßig 1 bis 30 und insbesondere 5 bis 20 Gew.-Teilen, bezogen auf 100 Gew.-Teile der Matrix, eingesetzt werden.

Im Hinblick auf Beispiele für geeignete **Verbindungen d5)** sei ausdrücklich auf Seite 7, Zeile 22 bis Seite 25, Zeile 21, der oben zitierten EP-A-768 336 verwiesen. Die dort genannten sterisch gehinderten Amine werden in die Offenbarung der vorliegenden Erfindung ausdrücklich mit einbezogen.

Im Hinblick auf Beispiele für geeignete **Verbindungen d6)** sei ausdrücklich auf Seite 27, Zeile 17 bis Seite 28, Zeile 9, der oben zitierten EP-A-768 336 verwiesen. Die dort genannten Dawsonite werden in die Offenbarung der vorliegenden Erfindung ausdrücklich mit einbezogen.

Im Hinblick auf Beispiele für geeignete **Verbindungen d7)** sei ausdrücklich auf Seite 28, Zeile 54 bis Seite 29, Zeile 6, der oben zitierten EP-A-768 336 verwiesen. Die dort genannten Alkali- und Erdalkaliverbindungen werden in die Offenbarung der vorliegenden Erfindung ausdrücklich mit einbezogen.

Im Hinblick auf Beispiele für geeignete **Verbindungen d8)** sei ausdrücklich auf Seite 31, Zeile 34 bis Seite 33, Zeile 4, der oben zitierten EP-A-768 336 verwiesen. Die dort genannten Antioxidantien werden in die Offenbarung der vorliegenden Erfindung ausdrücklich mit einbezogen.

Bezüglich der **Substanzen der Gruppe (d9)** sei ausdrücklich festgestellt, daß sowohl Gleitmittel als auch Trennmittel, als auch Gemische von Gleit- und Trennmitteln eingesetzt werden können. Nach dem üblichen Sprachgebrauch des Fachmanns bezeichnet man solche Produkte als Trennmittel, die die Reibungswiderstände überwiegend zwischen Polymerschmelze und Stahloberfläche der bei der formgebenden Verarbeitung eingesetzten Maschine reduzieren; die Reduktion des Reibungswiderstandes hat zur Folge, daß der Massedruck der Schmelze reduziert wird. Demgegenüber wirken Gleitmittel überwiegend in der Polymerschmelze und setzen die internen Reibungskräfte herab, wodurch die Schmelze auch bei hohen Füllstoffgehalten einen guten plastischen Fluß behält, der für die Ausfüllung des formgebenden Werkzeugs von Bedeutung ist.

In einer Ausführungsform der vorliegenden Erfindung werden als Gleit- bzw. Trennmittel bei 20°C feste oder flüssige Calciumsalze und/oder Magnesiumsalze und/oder Aluminiumsalze eingesetzt, die ausgewählt sind aus
- Calciumsalzen von gesättigten oder ungesättigten, geradkettigen oder verzweigten Monocarbonsäuren mit 6 bis 36 C-Atomen,
- Calciumsalzen der unsubstituierten oder mit C₁₋₄-Alkylresten substituierten Benzoesäure,
- Magnesiumsalzen von gesättigten oder ungesättigten, geradkettigen oder verzweigten Monocarbonsäuren mit 6 bis 36 C-Atomen,
- Magnesiumsalzen von gesättigten oder ungesättigten Dicarbonsäuren mit 6 bis 10 C-Atomen,
- Aluminiumsalzen von gesättigten oder ungesättigten, geradkettigen oder verzweigten Monocarbonsäuren mit 6 bis 36 C-Atomen

Für die vorstehend genannten Calcium-, Magnesium- und Aluminiumsalze gilt, daß sie sowohl allein als auch in Mischung eingesetzt werden könnten.

Weitere Gleit- bzw. Trennmittel, die alleine oder in Kombination miteinander als Komponente (d9) eingesetzt werden können, sind die hierfür einschlägig aus dem Stand der Technik bekannten Substanzen. Vorzugsweise kommen folgende Verbindungstypen in Frage: Kohlenwasserstoffwachse, die im Temperaturbereich von 70 bis 130°C schmelzen, oxidierte Polyethylenwachse, freie Fettsäuren mit 8 bis 22 C-Atomen und deren verzweigtkettige Isomere, beispielsweise Stearinsäure oder auch Hydroxystearinsäure, α-Olefine, Wachsester, d. h. Ester aus längerkettigen Monocarbonsäuren und Monoalkoholen, primäre und sekundäre, gesättigte und ungesättigte höhere Alkohole mit vorzugsweise 16 bis 44 C-Atomen im Molekül, Ethylendiamindistearat, Montansäureester von Diolen, beispielsweise von Ethandiol, 1,3-Butandiol und Glycerin, Mischungen derartiger Montansäureester mit unveresterten Montansäuren, Partialester aus Fettsäuren mit 8 bis 22 C-Atomen und Polyolen mit 2 bis 6 C-Atomen und 2 bis 6 Hydroxylgruppen, die pro Molekül im Durchschnitt mindestens eine freie Polyol-Hydroxylgruppe enthalten. Einsetzbar sind weiterhin die in der **DE-C-19 07 768** beschriebenen Mischester mit Hydroxyl- bzw. Säurezahlen im Bereich von 0 bis 6 aus aliphatischen, cycloaliphatischen oder aromatschen Dicarbonsäuren mit 2 bis 22 C-Atomen im Molekül, aliphatischen Polyolen mit 2 bis 6 Hydroxylgruppen im Molekül und aliphatischen Monocarbonsäuren mit 12 bis 30 C-Atomen im Molekül. Beispiele hierfür sind Mischester aus Maleinsäure-Pentaerythrit-Behensäure, Mischester aus Adipinsäure-Pentaerythrit-Ölsäure und Mischester aus Adipinsäure-Pentaerythrit-Stearinsäure. Derartige Gleit- oder Trennmittel können im Rahmen der vorliegenden Erfindung sowohl einzeln, als auch in Kombination miteinander, sowie auch in Kombination mit dem oben genannte Calcium-, Magnesium- oder Aluminiumsalzen eingesetzt werden.

Im Hinblick auf Beispiele für geeignete **Verbindungen d10)** sei ausdrücklich auf Seite 29, Zeile 20 bis Seite 30, Zeile 26, der oben zitierten EP-A-768 336 verwiesen. Die dort genannten Weichmacher werden in die Offenbarung der vorliegenden Erfindung ausdrücklich mit einbezogen.

Im Hinblick auf Beispiele für geeignete **Verbindungen d11)** sei ausdrücklich auf Seite 30, Zeile 28 bis Seite 30, Zeile 35, der oben zitierten EP-A-768 336 verwiesen. Die dort genannten Pigmente werden in die Offenbarung der vorliegenden Erfindung ausdrücklich mit einbezogen. Titandioxid ist als Pigment d11) bevorzugt.

Im Hinblick auf Beispiele für geeignete **Verbindungen d12)** sei ausdrücklich auf Seite 30, Zeile 37 bis Seite 30, Zeile 43, der oben zitierten EP-A-768 336 verwiesen. Die dort genannten Füllstoffe werden in die Offenbarung der vorliegenden Erfindung ausdrücklich mit einbezogen. Titandioxid ist als Pigment d11) bevorzugt. Bei den Füllstoffen d12) sind Calciumcarbonat (Kreide), Talkum, Kaolin und dergleichen bevorzugt. Kreide ist dabei ganz besonders bevorzugt.

Im Hinblick auf Beispiele für geeignete **Verbindungen d13)** sei ausdrücklich auf Seite 30, Zeile 45 bis Seite 31, Zeile 3, der oben zitierten EP-A-768 336 verwiesen. Die dort genannten Phosphite werden in die Offenbarung der vorliegenden Erfindung ausdrücklich mit einbezogen.

Im Hinblick auf Beispiele für geeignete **Verbindungen d14)** sei ausdrücklich auf Seite 31, Zeile 5 bis Seite 31, Zeile 19, der oben zitierten EP-A-768 336 verwiesen. Die dort genannten Thiophosphite und Thiophosphate werden in die Offenbarung der vorliegenden Erfindung ausdrücklich mit einbezogen.

Im Hinblick auf Beispiele für geeignete **Verbindungen d15)** sei ausdrücklich auf Seite 31, Zeile 21 bis Seite 31, Zeile 25, der oben zitierten EP-A-768 336 verwiesen. Die dort genannten Mercaptocarbonsäureester werden in die Offenbarung der vorliegenden Erfindung ausdrücklich mit einbezogen.

Im Hinblick auf Beispiele für geeignete **Verbindungen d16)** sei ausdrücklich auf Seite 31, Zeile 27 bis Seite 31, Zeile 32, der oben zitierten EP-A-768 336 verwiesen. Die dort genannten epoxidierten Fettsäureester werden in die Offenbarung der vorliegenden Erfindung ausdrücklich mit einbezogen.

Im Hinblick auf Beispiele für geeignete **Verbindungen d17)** sei ausdrücklich auf Seite 33, Zeile 6 bis Seite 34, Zeile 7, der oben zitierten EP-A-768 336 verwiesen. Die dort genannten UV-Absorber und Lichtschutzmittel werden in die Offenbarung der vorliegenden Erfindung ausdrücklich mit einbezogen.

Im Hinblick auf Beispiele für geeignete **Verbindungen d18)** sei ausdrücklich auf Seite 35, Zeile 9 bis Seite 35, Zeile 12, der oben zitierten EP-A-768 336 verwiesen. Die dort genannten Treibmittel werden in die Offenbarung der vorliegenden Erfindung ausdrücklich mit einbezogen.

Im Hinblick auf **Verbindung d19)** sei angemerkt, daß es sich hier um Harnstoff handelt und mithin um eine dem Fachmann bekannte Substanz definierter Struktur.

Ein weiterer Gegenstand der Erfindung ist die **Verwendun**g der nach dem erfindungsgemäßen Verfahren erhältlichen Aminouracile zur Stabilisierung von halogenhaltigen organischen Kunststoffen gegen photochemischen und/oder thermischen Abbau.

### Beispiele

### Zu den eingesetzten Substanzen

Evipol SH 6030 = S-PVC (k-Wert = 60)
Hydrocarb 95 T = Kreide
Sasil A 40 = handelsüblicher Zeolith
Loxiol G 22 = handelsübliches Gleitmittel (Fa. Cognis / DE)
Loxiol VPG 2571 = handelsübliches Gleitmittel (Fa. Cognis / DE)
Loxiol P 728 = handelsübliches Gleitmittel (Fa. Cognis / DE)
Loxiol P 2623 = handelsübliches Gleitmittel (Fa. Cognis / DE)
Omyalite 50 H = Kreide (Calciumcarbonat)
Vestowax A 227 = handelsübliches Paraffinwachs (Fa. Cognis / DE)
Dimethylaminouracil (gemäß Formel in der Beschreibung) als handelsübliches Produkt
N,N'-Dimethyl-N-cyanacetylharnstoff = handelsübliche Substanz in Form einer 80%igen Lösung in Wasser

### Beispiel 1

### (erfindungsgemäßes Verfahren)

In einem Henschelmischer (für maximal 2 kg Einwaage) wurden insgesamt 1 kg einer Mischung der in Tabelle 1 aufgeführten Komponenten mit Ausnahme der Komponente "N,N'-Dimethyl-N-cyanacetylharnstoff" gegeben:

**Tabelle 1:**

| **Komponente** | **Gew.-%** |
|---|---|
| Sasil A 40 | 35,71 |
| N,N'-Dimethyl-N-cyanacetylharnstoff (*) | 8,5 |
| Natriumperchlorat (**) | 4,46 |
| Calciumstearat | 13,39 |
| Loxiol VPG 2571 | 26,79 |
| Loxiol G 22 | 4,46 |
| Vestowax A 227 | 4,46 |
| Calciumhydroxid | 2,23 |
| Summe: | **100,0** |

| | |
|---|---|
| N,N'-Dimethyl-N-cyanacetylharnstoff (*) -> in Form einer 80%igen Lösung in Wasser Natriumperchlorat | |
| (**) -> in Form einer 50%igen Lösung in Wasser | |

Diese Mischung wurde bei 800 Umdrehungen pro Minute ca. 30 Sekunden lang homogenisiert. Anschließend wurde die in Tabelle 1 angegebene Komponente Komponente "N,N'-Dimethyl-N-cyanacetylhamstoff" in der dort angegebenen Menge zudosiert und die Mischung bei 800 Umdrehungen pro Minute ca. 4 ½ Minuten vermischt. Unmittelbar anschließend wurde noch eine weitere Minute bei 1200 Umdrehungen pro Minute gemischt. Das erhaltene Compound wurde in Beispiel 7 eingesetzt (siehe Tabelle 2).

### Beispiele 2 bis 7 ( B 2 bis B 7)

### (Anwendungsbeispiele)

In der untenstehenden Tabelle 2 wurden einerseits die einzelnen Rezepturbestandteile der untersuchten Prüfrezepturen angegeben, andererseits die ermittelten Prüfergebnisse dargestellt. In der ersten Zeile der Tabelle sind die jeweiligen Versuchsnummern angegeben. Die Mengenanteile der einzelnen Komponenten sind in phr angegeben; phr bedeutet dabei "part per hundred resin" und gibt an, wieviele Gewichtsteile der jeweiligen Komponente nach der Zugabe der Zusammensetzung im PVC - bezogen auf 100 Gewichtsteile PVC - vorhanden sind. Dementsprechend enthalten die Rezepturen jeweils 100 Teile PVC (Evipol SH 6030).

Bei den Prüfrezepturen wurden jeweils drei Messungen durchgeführt:
• **Stabilitätstest bei thermischer Belastung:** Entsprechend den Rezepturen wurden Walzfelle hergestellt und die statische Thermostabilität bei 180°C bestimmt. Die Herstellung der Walzfelle erfolgte, indem man die PVC-Pulvermischung und die genannten Rezepturkomponenten auf einem Laborwalzwerk 5 Minuten lang bei 170 °C homogenisierte und plastifizierte. Aus den so hergestellten etwa 0,5 mm dicken Walzfellen wurden Teststücke (Prüfkörper) der Größe von 17 x 17 mm herausgeschnitten. Die Prüfkörper wurden bei 180 °C im Wärmeschrank auf Glasplatten auf rotierenden Horden plaziert und in 15-minütigen Abständen wieder entnommen, bis alle Proben "verbrannt" waren (d.h. Schwarzfärbung erreicht hatten)
• **Farbmessung am Walzfell:** Darüber hinaus wurde bei den Walzfellen zur weiteren Charakterisierung die dem Fachmann bekannte L*,a*,b*-Methode (vergleiche DIN 6174) herangezogen. Der L-Wert gibt dabei die Helligkeit an. Bei den Messungen kam ein handelsübliches Gerät mit der Bezeichnung "Micro Color" (Firma Dr. Lange) zum Einsatz.
• **Stabilitätstest nach der Kongorot-Methode:** Weiterhin wurden bei den Walzfellen zur weiteren Charakterisierung die dem Fachmann bekannte Kongorot-Methode gemäß der Euro-Norm EN 60811-3-2:1995 Abs.9 herangezogen. Hierzu wurden von den Walzfellen jeweils kleine Proben (50 ±5 mg) entnommen und in den entsprechenden Glasröhrchen in einem Metallblock auf 200°C ( ±0.5 °C) erwärmt. In das obere Ende des Glasröhrchen wurde ein Streifen Universalindikatorpapier eingeführt. Die Zeit, bis die Farbe des Indikatorpapiers gerade nach rot umschlug, wurde in Minuten gemessen.

**Tabelle 2:**

| | B2 | B3 | B4 | B5 | B6 | **B7** | | | |
|---|---|---|---|---|---|---|---|---|---|
| **Rezepturen - Angabe der Bestandteile in phr** | | | | | | | | | |
| Evipol SH 6030 | 100 | 100 | 100 | 100 | 100 | 100 | | | |
| Hydrocarb 95 T | 1 | 1 | 1 | 1 | 1 | 1 | | | |
| Sasil A 40 | 0,8 | 0,8 | 0,8 | 0,8 | 0,8 | | | | |
| Natriumperchlorat (**) | 0,1 | 0,1 | 0,1 | 0,1 | 0,1 | | | | |
| Calciumstearat | 0,3 | 0,3 | 0,3 | 0,3 | 0,3 | | | | |
| Loxiol VPG 2571 | 0,6 | 0,6 | 0,6 | 0,6 | 0,6 | | | | |
| Loxiol G 22 | 0,1 | 0,1 | 0,1 | 0,1 | 0,1 | | | | |
| Vestowax A 227 | 0,1 | 0,1 | 0,1 | 0,1 | 0,1 | | | | |
| Calciumhydroxid | | | 0,05 | | 0,05 | | | | |
| Dimethylaminouracil | | | | 0,15 | 0,15 | | | | |
| N,N'-Dimethyl-N-cyanacetylharnstoff (*) | | 0,19 | 0,19 | | | | | | |
| Compound gemäß Beispiel 1 | | | | | | 2,24 | | | |

| **Farbmessung am Walzfell - Helligkeit \ [L]** | | | | | | | | | |
|---|---|---|---|---|---|---|---|---|---|
| ungelagert | 72 | 77 | 79 | 81 | 80 | 82 | | | |
| 15 min. bei T=180 °C | 63 | 73 | 75 | 78 | 76 | 80 | | | |
| 30 min bei T= 180 °C | 60 | 59 | 63 | 71 | 71 | 75 | | | |

| **Stabilitätsende** **nach Lagerung im Ofen (T=180 °C)** | | | | | | | | | |
|---|---|---|---|---|---|---|---|---|---|
| [min ] | 60 | 60 | 60 | 60 | 60 | 90 | | | |

| **Stabilitätsende** **gemäß Kongorot (T=200 °C)** | | | | | | | | | |
|---|---|---|---|---|---|---|---|---|---|
| [min] | 11 | 12 | 14 | 14 | 15 | 16 | | | |

• die Beispiele 2 bis 6 dienen zum Vergleich, Beispiel 7 ist erfindungsgemäß
• Dimethylaminouracil (*) -> gemäß der Formel in der Beschreibung; komerziell erhältliches Produkt
• N,N'-Dimethyl-N-cyanacetylharnstoff (*) -> in Form einer 80%igen Lösung in Wasser
• Natriumperchlorat (**) -> in Form einer 50%igen Lösung in Wasser

### Beispiel 8

### (erfindungsgemäßes Verfahren)

180 g Loxiol VPG 2571 wurden bei 80 °C mit 15 g Calciumhydroxid versetzt. Zu dieser Suspension wurden 105 g einer 80 %igen wäßrigen Lösung N,N'-Dimethyl-N-cyanacetylharnstoff in Wasser gleichmäßig über einen Zeitraum von 5 Minuten zugetropft. Die Suspension wurde für 15 Minuten auf 120 °C erwärmt und das enthaltene Wasser im Vakuum entfernt. Man erhielt ca. 280 g einer feinverteilten Suspension, die als Pastille, Schuppe oder Sprühprodukt konfektioniert werden kann.

### Beispiel 9

### (erfindungsgemäßes Verfahren)

Beispiel 8 wurde wiederholt, wobei
- anstelle von 180 g Loxiol VPG 2571 als Gleitmittel 180 g Dea Wax LS 567 und
- anstelle von 15 g Calciumhydroxid als basische Verbindung 15 g NaOH eingesetzt wurden und die Reaktionstemperatur statt auf 120 °C auf 90 °C eingestellt wurde.

### Beispiel 10

### (erfindungsgemäßes Verfahren)

Beispiel 8 wurde wiederholt, wobei
- anstelle von 180 g Loxiol VPG 2571 als Gleitmittel 180 g Loxiol P 728 eingesetzt wurde und die Reaktionstemperatur statt auf 120 °C auf 90 °C eingestellt wurde.

### Beispiel 11

### (erfindungsgemäßes Verfahren)

Beispiel 8 wurde wiederholt, wobei
- anstelle von 180 g Loxiol VPG 2571 als Gleitmittel 171 g Zinklaurat und
- anstelle von 105 g einer 80 %igen wäßrigen Lösung N,N'-Dimethyl-N-cyanacetylharnstoff in Wasser als Verbindung (i) 40g wasserfreies N,N'-Dimethyl-N-cyanacetylharnstoff
   eingesetzt wurde und die Reaktionstemperatur statt auf 120 °C auf 140 °C eingestellt wurde.

### Beispiel 12

### (erfindungsgemäßes Verfahren)

Beispiel 8 wurde wiederholt, wobei
- anstelle von 180 g Loxiol VPG 2571 als Gleitmittel eine Mischung von 180 g Loxiol P 2623 und 21 g Wasser und
- anstelle von 105 g einer 80 %igen wäßrigen Lösung N,N'-Dimethyl-N-cyanacetylharnstoff in Wasser als Verbindung (i) 84g wasserfreies N,N'-Dimethyl-N-cyanacetylharnstoff eingesetzt wurde und die Reaktionstemperatur statt auf 120 °C auf 90 °C eingestellt wurde.

### Beispiele 13 und 14 (B 13 und B 14)

### (Anwendungsbeispiele)

Aus PVC-Pulver und verschiedenen Additiven wurde in einem Mischer der Firma Henschel ein Dry-Blend hergestellt (Materialmenge = 3 kg, Heiztemperatur = 120°C, anschließendes Abkühlen); die Zusammensetzungen sind Tabelle 3 zu entnehmen. Das Dry-Blend wurde auf einem Doppelschneckenextruder der Firma. Weber zu einem Flachband extrudiert. (Parameter der Extrusion: Drehzahl = 15 UpM; Maschinenbelastung = 42%, Temperatur = 180°C). Von den so extrudierten Flachbändern wurde die statische Thermostabilität bei 180°C bestimmt (visuelle Beurteilung). Die Ergebnisse sind Tabelle 4 zu entnehmen.

**Tabelle 3:**

| **Zusammensetzungen (stabilisiertes PVC)** | | |
|---|---|---|
| Die Bestandteile der Rezeptur sind (analog zu Tabelle 2) in phr angegeben | | |

| Beispiel: | **B13** | **B14** |
|---|---|---|
| Evipol SH 6830 | 100 | 100 |
| Omyalite 50 H | 6 | 6 |
| Zeolith A | 0.7 | 0.7 |
| 1,3-Dimethyl-4-aminouracil | 0.26 | - |
| Loxiol VPG 2571 | 0.55 | - |
| **Compound nach Beispiel 8** | - | 0.86 |
| Na-Perchlorat, 50%-ige Lösung in Wasser | 0.09 | 0.09 |
| Magnesiumstearat | 0.26 | 0.26 |
| Loxiol P 2518 | 0.1 | 0.1 |

**Tabelle 4:**

| **Statische Themostabilität** | | | | |
|---|---|---|---|---|
| Beispiel | nach 15 Min | nach 30 Min | Stabilitätsende | |
| | Farbe | Farbe | Farbe | Zeit [Min] |
| B 13 | beige | leicht gelb | leicht gelb | 105 |
| B 14 | beige | leicht gelb | leicht gelb | 105 |

## Patentansprüche

1. **Verfahren** zur Herstellung von Aminouracilen der Formel (ii) 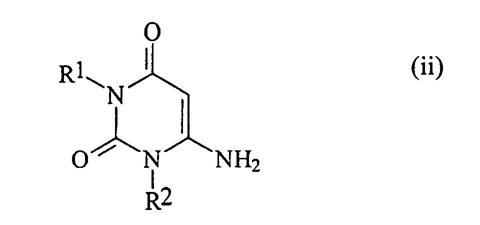 worin die Reste R¹ und R² unabhängig voneinander jeweils Wasserstoff oder einen unverzweigten oder verzweigten, linearen oder cyclischen Alkylrest mit 1 bis 18 C-Atomen oder einen Arylrest mit 6 bis 18 C-Atomen, der gegebenenfalls durch ein oder mehrere Alkylreste mit jeweils 1 bis 6 C-Atomen substituiert sein kann, bedeuten,
durch Umsetzung der entsprechenden Cyanacetylharnstoffe der Formel (i) 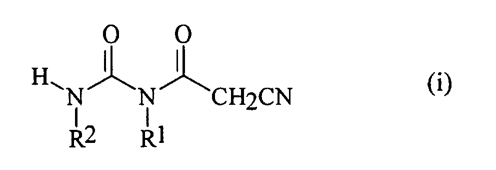 worin die Reste R¹ und R² die oben genannte Bedeutung haben,
mit basischen Verbindungen, wobei man die Umsetzung in einer Matrix durchführt, mit der Maßgabe, daß diese Matrix eine Zusammensetzung darstellt, die ein oder mehrere Verbindungen enthält, die ausgewählt sind aus der Gruppe der Kunststoffadditive, der Kunststoffstabilisatoren und der halogenhaltigen organischen Kunststoffe.

2. Verfahren nach Anspruch 1, wobei die Reste R¹ und R² unabhängig voneinander jeweils einen unverzweigten oder verzweigten, linearen oder cyclischen Alkylrest mit 1 bis 18 C-Atomen bedeuten.

3. Verfahren nach Anspruch 1 oder 2, wobei die Matrix frei ist von halogenhaltigen organischen Kunststoffen.

4. Verfahren nach einem der Ansprüche 1 bis 3, wobei man die basischen Verbindungen auswählt aus der Gruppe der Alkali- und Erdalkalihydroxide.

5. Verfahren nach einem der Ansprüche 1 bis 4, wobei man die Umsetzung in einem Mischer durchführt.

6. Verfahren nach einem der Ansprüche 1 bis 5, wobei man die Umsetzung in einem Extruder durchführt.

7. Verfahren nach einem der Ansprüche 1 bis 6, wobei der Wassergehalt der Matrix unterhalb von 10 Gew.-% - bezogen auf die gesamte Matrix - beträgt.

8. Verfahren nach einem der Ansprüche 1 bis 7, wobei die Matrix aus ein oder mehreren Gleitmitteln besteht.

9. Verfahren nach Anspruch 8, wobei man die Gleitmitteln zunächst aufschmilzt, mit Alkali und/oder Erdalkalihydroxiden versetzt und die Komponente (i) zu der dabei erhaltenen Suspension gleichmäßig zudosiert, vorzugsweise in Form einer wäßrigen Lösung

10. Verwendung der nach dem Verfahren gemäß den Ansprüchen 1 bis 9 erhältlichen Aminouracile zur Stabilisierung von halogenhaltigen organischen Kunststoffen gegen photochemischen und/oder thermischen Abbau.

## Claims

1. A process for the production of aminouracils corresponding to formula (ii): 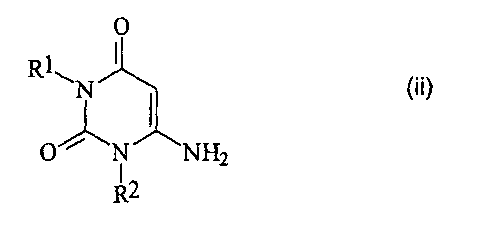 in which R¹ and R² independently of one another represent hydrogen or an unbranched or branched, linear or cyclic alkyl group containing 1 to 18 carbon atoms or an aryl group containing 6 to 18 carbon atoms, which may optionally be substituted by one or more alkyl groups containing 1 to 6 carbon atoms,
by reaction of the corresponding cyanoacetyl ureas corresponding to formula (i): 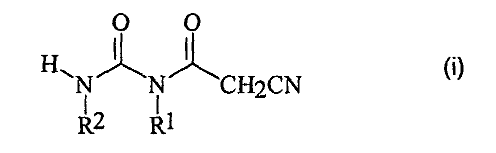 in which R¹ and R² are as defined above,
with basic compounds, the reaction being carried out in a matrix, with the proviso that this matrix is a composition which contains one or more compounds selected from the group of additives for plastics, stabilizers for plastics and halogen-containing organic plastics.

2. A process as claimed in claim 1, **characterized in that** R¹ and R² independently of one another represent an unbranched or branched, linear or cyclic alkyl group containing 1 to 18 carbon atoms.

3. A process as claimed in claim 1 or 2, **characterized in that** the matrix is free from halogen-containing organic plastics.

4. A process as claimed in any of claims 1 to 3, **characterized in that** the basic compounds are selected from the group of alkali metal and alkaline earth metal hydroxides.

5. A process as claimed in any of claims 1 to 4, **characterized in that** the reaction is carried out in a mixer.

6. A process as claimed in any of claims 1 to 5, **characterized in that** the reaction is carried out in an extruder.

7. A process as claimed in any of claims 1 to 6, **characterized in that** the water content of the matrix is below 10% by weight, based on the matrix as a whole.

8. A process as claimed in any of claims 1 to 7, **characterized in that** the matrix consists of one or more lubricants.

9. A process as claimed in claim 8, **characterized in that** the lubricants are first melted, alkali metal and/or alkaline earth metal hydroxides are added and component (i) is uniformly introduced into the suspension obtained, preferably in the form of an aqueous solution.

10. The use of the aminouracils obtainable by the process claimed in claims 1 to 9 for stabilizing halogen-containing organic plastics against photochemical and/or thermal degradation.

## Revendications

1. Procédé de production d'aminouraciles de formule (ii) 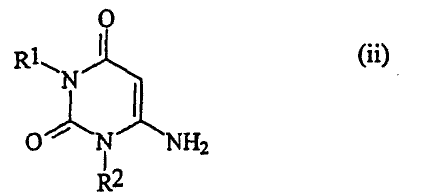 dans laquelle
les restes R¹ et R² indépendamment l'un de l'autre, signifient respectivement de l'hydrogène, ou un reste alkyle non ramifié ou ramifié, linéaire ou cyclique ayant de 1 à 18 atomes de carbone, ou un reste alkyle ayant de 6 à 18 atomes de carbone, qui peut être substitué le cas échéant par un ou plusieurs reste(s) alkyle(s) ayant respectivement de 1 à 6 atomes de carbone,
par mise en réaction de cyanacetylurées correspondantes de formule (i) 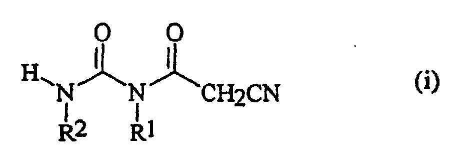 dans laquelle les restes R¹ et R² possèdent la signification mentionnée ci-dessus avec des composés basiques, condensation dans laquelle on effectue la réaction dans une matrice, avec la précision que cette matrice représente une composition qui renferme un ou plusieurs composé(s) qui sont choisis dans le groupe des additifs de matières plastiques, des agents stabilisants pour matière plastique et des matières plastiques organiques contenant des halogènes.

2. Procédé selon la revendication 1,
dans lequel
les restes R¹ et R² indépendamment l'un de l'autre, signifient respectivement un reste alkyle non ramifié ou ramifié, linéaire ou cyclique, ayant de 1 à 18 atomes de carbone.

3. Procédé selon la revendication 1 ou 2,
dans lequel
la matrice est dépourvue de matière plastique organique contenant des halogènes.

4. Procédé selon l'une des revendications 1 à 3,
dans lequel
on sélectionne les composés basiques, dans le groupe des hydroxydes de métal alcalin et des hydroxydes de métal alcalino-terreux.

5. Procédé selon l'une des revendications 1 à 4,
dans lequel
on effectue la réaction dans un mélangeur.

6. Procédé selon l'une des revendications 1 à 5,
dans lequel
on effectue la réaction dans une extrudeuse.

7. Procédé selon l'une des revendications 1 à 6,
dans lequel
la teneur en eau de la matrice s'élève à en dessous de 10% en poids - rapporté à la matrice totale.

8. Procédé selon l'une des revendications 1 à 7,
dans lequel
la matrice consiste en un ou plusieurs agents de glissement.

9. Procédé selon la revendication 8,
dans lequel
on fait fondre en premier lieu les agents de glissement, on additionne d'hydroxydes de métal alacalin et/ou alcalino-terreux et on ajoute par doses d'une manière régulière le composant (i) à la suspension ainsi obtenue, de préférence sous la forme d'une solution aqueuse.

10. Utilisation des aminouraciles accessibles selon le procédé conformément aux revendications 1 à 9, en vue de la stabilisation de matières plastiques organiques contenant des halogènes, contre la dégradation photochimique et/ou thermique.
